# EUROPÄISCHE PATENTSCHRIFT

(11) **EP 1 769 004 B1**
(45) Veröffentlichungstag und Bekanntmachung des Hinweises auf die Patenterteilung: **09.08.2017**
(21) Anmeldenummer: 05768238.7
(22) Anmeldetag: 20.06.2005
(51) Int. Cl.: C08B 15/00, A61L 15/28, A61L 15/60

(54) **WASSERABSORBIERENDES POLYSACCHARID SOWIE EIN VERFAHREN ZU SEINER HERSTELLUNG**
WATER-ABSORBING POLYSACCHARIDE AND METHOD FOR PRODUCING THE SAME
POLYSACCHARIDE ABSORBANT L'EAU ET PROCEDE DE PRODUCTION ASSOCIE

(30) Priorität: 21.06.2004 DE 102004029713; 22.06.2004 DE 102004030182; 24.03.2005 DE 102005013893
(43) Veröffentlichungstag der Anmeldung: 04.04.2007
(73) Patentinhaber: Evonik Degussa GmbH, 45128 Essen (DE)
(72) Erfinder: FRANK, Markus, 76532 Baden-Baden (DE); LOEKER, Frank, 47798 Krefeld (DE); PAEPEN, Dirk, 47608 Geldern (DE); SMITH, Scott, Greensboro, NC 27407 (US)
(74) Vertreter: Lang, Arne
(86) Internationale Anmeldenummer: PCT/EP2005/006619
(87) Internationale Veröffentlichungsnummer: WO 2005/123781

(56) Entgegenhaltungen:
- EP-A- 0 699 793
- EP-A- 0 900 807
- WO-A-98/27117
- WO-A-03/022887
- WO-A-2004/085481
- DE-A1- 2 543 187
- DE-A1- 4 013 047
- DE-A1- 4 033 007
- DE-A1- 19 654 745
- DE-A1- 19 729 272
- W.-M. KULICKE, Y.A. AGGOUR, M.Z. ELSABEE: "Preparation, Characterisation, and Rheological Behaviour of Starch-Sodium Trimetaphosphate Hydrogels" STARCH/STÄRKE, Bd. 42, Nr. 4, 1990, Seiten 134-141, XP009053530

## Beschreibung

Die vorliegende Erfindung betrifft allgemein ein Verfahren zur Herstellung eines wasserabsorbierenden Polysaccharids, ein durch dieses Verfahren erhältliches wasserabsorbierendes Polysaccharid, einen Verbund, ein Verfahren zur Herstellung eines Verbundes, einen durch dieses Verfahren hergestellten Verbund, die Verwendung der wasserabsorbierenden Polysaccharide oder der Verbunde.

Die meisten der heute verwendeten Absorptionsmaterialien, die in der Lage sind, in kurzer Zeit große Flüssigkeitsmengen (Wasser, Urin) aufzunehmen, stellen in erster Linie schwach vernetzte, synthetische Polymere dar. Dazu zählen beispielsweise Polymere und Copolymere auf Basis von Acrylsäure oder Acrylamid, die nicht auf nachwachsenden Rohstoffen basieren und unzureichend bzw. überhaupt nicht biologisch abbaubar sind.

Im Stand der Technik werden jedoch zahlreiche wasserabsorbierende Polymere beschrieben, die auf Polysacchariden basieren und die zumindest teilweise biologisch abbaubar sind. Die Rohstoffe zur Herstellung von Superabsorbern auf Polysaccharidbasis sind jedoch häufig wasserlöslich und müssen in die wasserunlösliche Form überführt werden, um sie als Superabsorber für Hygieneanwendungen verwenden zu können.

So beschreiben EP 0 538 904 A1 bzw. US 5,247,072 Superabsorber auf Basis von Carboxyalkylpolysacchariden. In den Verfahren wird das Carboxyalkylpolysaccharid in Wasser gelöst und durch Trocknung oder Fällung isoliert und anschließend thermisch durch die Reaktion der Hydroxylgruppen des Polysaccharidskeletts mit den sauren Carboxylgruppen über interne Esterbrücken vernetzt. Da diese Vernetzungsreaktion sehr empfindlich auf geringe Änderungen des pH-Wertes, der Temperatur oder der Reaktionsdauer reagiert, werden Absorber mit stark schwankenden Absorptionseigenschaften erhalten. Die Materialien zeichnen sich durch ein hohes Absorptionsvermögen unter Druck aus, welches jedoch bei Lagerung der Absorber innerhalb weniger Wochen auf einen Bruchteil des ursprünglichen Aufnahmevermögens abfällt.

In der US 5,550,189 werden Absorber auf Basis von Carboxyalkylpolysacchariden beschrieben, bei denen die Alterungsstabilität durch Zusatz von mehrfachfunktionellen Vernetzern wie z.B. Aluminiumsalze oder Citronensäure verbessert wird. Die Herstellung der Absorber erfolgt aus einer gemeinsamen, homogenen wässrigen Lösung von Carboxyalkylpolysaccharid und Vernetzer, in der die Komponenten in geringer Konzentration vorliegen, gemeinsam isoliert und dann thermisch vernetzt werden. Die Synthese dieser Absorber erfordert einen hohen Energie- und Zeitaufwand, da die wässrigen Lösungen nur sehr niedrig konzentriert sind. Die Verbesserung der Alterungsstabilität entspricht bei der Mehrzahl der Ausführungsbeispiele nicht den praxisrelevanten Anforderungen.

Die EP 855 405 A1 behandelt das Problem der mangelnden Alterungsbeständigkeit des Absorptionsvermögens quellbarer Stärkemaleate und schlägt als Lösung eine Anlagerung von Mercaptoverbindungen an die Doppelbindung des Maleinsäuresubstituenten vor. Das Absorptionsverhalten der Produkte, insbesondere unter Druckbelastung, ist sehr gering.

In der US 4,952,550 wird die Herstellung eines auf Carboxymethylcellulose basierenden Absorbers beschrieben, wobei die Carboxymethylcellulose in Wasser oder org. Lösemittel mit mehrwertigen Metallsalzen und einer hydrophobierenden Komponente behandelt wird. Eine thermische Vernetzung erfolgt nicht. Gemäss Offenbarung wird das Gelblocking bei diesen Absorbern durch die hydrophobierende Komponente gemindert.

Bei den aus dem Stand der Technik bekannten Verfahren zur Vernetzung von Polysacchariden wurde jedoch neben der teilweise geringen Alterungsbeständigkeit beobachtet, dass die homogene Vernetzung der Polysaccharide die biologische Abbaubarkeit des Absorbers behindert, da die Zugänglichkeit für Mikroorganismen durch die eingeschränkte Quellung vermindert wird. Darüber hinaus wird bei den aus dem Stand der Technik bekannten Vernetzungsreaktionen der enzymatische Abbau durch die zusätzlich eingeführten Substituenten gehemmt [Mehltretter et al., Journal of the American Oil Chemists Society, 47(1970)Seite 522-524].

Um diese nachteiligen Eigenschaften zu verbessern, wurde vorgeschlagen, die Vernetzung des Polysaccharids auf den Oberflächenbereich zu beschränken, was jedoch in der Regel zu Produkten führt, die zwar eine zufriedenstellende Absorption unter Druck aufweisen, häufig jedoch nur durch eine unbefriedigende Absorptionskapazität unter Normaldruck und vor allem, bedingt durch die Beschränkung der Vernetzung auf den Oberflächenbereich, durch eine im Vergleich zu homogen vernetzten Polymeren geringe Gelfestigkeit gekennzeichnet sind. Geringe Gelfestigkeiten führen zur Bildung von Feinstaubanteilen bei Verarbeitungsprozessen, wie beispielsweise dem Sieben oder der Förderung, und somit zu Gesundheitsbelastungen des mit der Herstellung der Superabsorber beauftragten Arbeitspersonals.

So beschreibt WO 02/096953 A1 ein Verfahren zur Herstellung von Superabsorbern auf Basis oberflächenmodifizierter Polycarboxypolysaccharide, bei dem ein unvernetztes Polysaccharid mit Wasser unter Bildung eines Hydrogels gequollen wird, das Hydrogel anschließend mechanisch zerkleinert und getrocknet wird und anschließend die so erhaltenen Polymerpartikel mit einer Lösung eines Vernetzers beschichtet und einer Oberflächenvernetzung unterworfen werden. Nachteilig bei dem in WO 02/096953 A1 beschriebenen Verfahren ist jedoch, dass bei der Bildung des Hydrogels dem Wasser ein organisches Lösungsmittel zugesetzt werden muss, um die Quellung des Polysaccharids zu fördern. Der Zusatz des organischen Lösungsmittels führt jedoch dazu, dass die gequollenen Polysaccharide äußerst "schleimig" sind, was ihre Weiterverarbeitung deutlich erschwert. Außerdem verbleiben die organischen Lösungsmittel zumindest teilweise im Endprodukt, was aus ökologischen Gründen bedenklich ist. Auch die WO 00/21581 A1 offenbart ein Verfahren, bei dem Gele aus vernetzten Polysacchariden mit organischen Lösungsmitteln in Kontakt gebracht werden, um so absorbierende Polysaccharide mit verbesserten Absorptionseigenschaften zu erhalten. Nachteilig ist auch bei diesem Verfahren vor allem der Einsatz organischer Lösungsmittel.

US 5,470,964 beschreibt die Herstellung eines an der Oberfläche mit mehrwertigen Metallionen vernetzten Absorbers auf Basis säuregruppenhaltiger Polysaccharide, der eine verbesserte Absorption gegen Druck aufweist. Nachteilig bei diesem Verfahren ist, dass für die verbesserte Aufnahmefähigkeit des Absorbers gegen Druck eine relativ dicke Schicht der Oberfläche vernetzt werden muss und dass dies gemäss Offenbarung nur durch vorheriges Anquellen des Polysaccharids mit großer Lösungsmittelmenge möglich ist. Im angequollenen Zustand können dann die mehrwertigen Metallionen tief genug in die Oberfläche eindringen. Um dies zu erreichen wird das Polysaccharid in einen Überschuss der wässrigen Metallsalzlösung gegeben wobei der Wasserüberschuss in Bezug auf das Polysaccharid bei der 2fachen bis 40fachen Menge liegt. Durch die dicke vernetzte Oberflächenschicht werden zwar gute Absorptionswerte gegen Druck erzielt, das freie Quellvermögen sowie das Retentionsvermögen des Absorbers wird dadurch jedoch nachteilig reduziert. Ferner ist bei dem beschriebenen Verfahren nachteilig, dass dem im Herstellungsprozess zuletzt in die Vernetzerlösung zugegebenen Teil des Polysaccharids weniger Quellzeit und eine geringere Vernetzerkonzentration zur Verfügung steht, so dass eine inhomogene Verteilung des Vernetzers auf der Oberfläche resultiert wodurch sich starke Schwankungen der Absorptionseigenschaften ergeben.

Allgemein lag der Erfindung die Aufgabe zugrunde, die sich aus dem Stand der Technik ergebenden Nachteile zu überwinden.

Aufgabe der vorliegenden Erfindung war es daher, biologisch abbaubare, superabsorbierende Polymere auf Basis nachwachsender Rohstoffe bereitzustellen, die die zuvor beschriebenen Mängel nicht aufweisen.

Insbesondere sollen die Absorber eine hohe Langzeitlagerstabilität aufweisen, in der die Absorptionseigenschaften möglichst weitgehend erhalten bleiben.

Gleichzeitig ist anzustreben, dass die Absorberpartikel eine hohe mechanische Stabilität aufweisen, um die Bildung von Feinstaubanteilen bei Verarbeitungsprozessen wie beispielsweise dem Sieben oder der Förderung zu vermeiden.

Ferner sollten die Absorber hinsichtlich des Absorptionsverhaltens insbesondere bei viel Superabsorber beinhaltenden Sauglagen (meist mehr als 65 Gew.-% bezogen auf die Sauglage) nicht zum Gelblocking neigen und neben einer hohen Absorptions- und Retentionskapazität auch eine hohe Aufnahmekapazität gegen Druck für Wasser und wässrige Lösungen besitzen.

Bei viel Superabsorber beinhaltenden Sauglagen bzw. Cores und den diese beinhaltenden Windeln ist oftmals ein als Leakage bezeichnetes Durchnässen zu beobachten. Dieses und das Gelblocking beruhen in der Regel auf einem schleimigen gequollenen Hydrogel oder zumindest auf schleimigen Bestandteilen des Hydrogels. Daher bestand eine Aufgabe dieser Erfindung darin, ein weniger schleimiges Hydrogel bildendes absorbierendes Polymer zur Verfügung zu stellen, das sich zur Verwendung in Hygieneartikeln eignet.

Für ein gutes Absorptions- und Anwendungsverhalten ist es erforderlich, dass die Absorber auch in einem Überschuss an wässriger Lösung einen überwiegend unlöslichen Charakter aufweisen. Weiterhin sollen die Absorber durch eine besonders gute biologische Abbaubarkeit gekennzeichnet sein und möglichst frei von organischen Lösungsmitteln sein.

Eine weitere Aufgabe der Erfindung ist es, ein Herstellungsverfahren für solche superabsorbierenden Polymere zu finden, das einfach, ökonomisch und sicher durchführbar ist, eine gleichmäßige Produktqualität liefert und bei dem niedrige Lösungsmittelmengen verwendet und organische Lösungsmittel nach Möglichkeit vermieden werden. Darüber hinaus sollen die Verfahren ohne die Verwendung toxikologisch bedenklicher Substanzen durchführbar sein.

Zudem besteht eine erfindungsgemäße Aufgabe darin, die biologische Abbaubarkeit von Hygieneartikeln wie Damenbinden, Wundauflagen, Inkontinenzartikeln und Windeln zu verbessern.

Einen Beitrag zur Lösung dieser Aufgaben leistet ein Verfahren zur Herstellung eines wasserabsorbierenden Polysaccharids nach Anspruch 1.

Bevorzugt erfolgt das in Kontakt bringen in einem Kneter. Hierbei ist es bevorzugt, dass in dem Kneter eine möglichst homogene und innige Vermischung des Vernetzungsmittels mit dem Polysaccharid erfolgt. Es ist bevorzugt, dass die Vernetzung in erster Linie in dem Trocknungsschritt erfolgt. Auch in dieser Ausgestaltung des erfindungsgemäßen Verfahrens ist das Vernetzungsmittel vorzugsweise ein Polyphosphat oder Polyphosphorsäure.

Es ist weiterhin in dem erfindungsgemäßen Verfahren bevorzugt, dass der Kneter mindestens zwei Knetwellen aufweist. Die mindestens zwei Knetwellen besitzen vorzugsweise eine mindestens teilweise ineinander greifende Kontur. Hierbei handelt es sich vorzugsweise um an der Knetwelle angebrachte Elemente wie Scheiben, Paddel, Anker oder Harken, die von der Zentralachse der Knetwelle aus gesehen Rotationsradien bilden, die sich mit den Rotationsradien der an einer weiteren Knetwelle angeordneten Elemente überschneiden. Dieses kann beispielsweise dadurch erreicht werden, dass die Knetwellen mindestens abschnittweise achsparallel zueinander angeordnet sind und an dem achsparallelen Abschnitt der Abstand der Zentralachsen der Knetwellen so klein gewählt ist, dass die auf den Knetwellen ausgebildeten Elemente sich bei Betrieb der Knetwellen mindestens teilweise überlappen. Es ist ferner in dem erfindungsgemäßen Verfahren bevorzugt, dass mindestens ein Teil der auf der Knetwelle ausgebildeten Elemente so angeordnet und ausgestaltet sind, dass diese das zu fördernde Gut mindestens teilweise parallel zur Zentralachse der Knetwelle fördern, wobei die mindestens zwei Knetwellen einen mindestens zu einer der Knetwellen mindestens teilweise axial verlaufenden Förderkanal ausbilden. So kann zum einen eine möglichst homogene Vermischung des Polysaccharids mit dem Vernetzungsmittel erreicht und die homogene, das Polysaccharid und das Vernetzungsmittel beinhaltende Mischung kontinuierlich dem durch Temperaturbehandlung erfolgenden Vernetzungs- bzw. Trockenschritt zugeführt werden.

Im Zusammenhang mit dem Homogenisieren ist es bevorzugt, dass in dem Kontakt-Schritt der Anteil an bereits abreagierten Vernetzungsmittel nicht über 30 Gew.-%, vorzugsweise nicht über 20 Gew.-% und besonders bevorzugt nicht über 10 Gew.-% sowie darüber hinaus bevorzugt nicht über 5 Gew.-%, jeweils bezogen auf das Vernetzungsmittel, liegt. Der Anteil des bereits abreagierten Vernetzungsmittel lässt sich durch Subtraktion des bestimmbaren freien Vernetzungsmittels von dem ursprünglich eingesetzten ermitteln.

In einer Ausgestaltung des erfindungsgemäßen Verfahrens findet das in Kontakt bringen von Polysaccharid und Vernetzungsmittel in einem Kneter statt, wohingegen der auf das in Kontakt bringen folgende Vernetzungs- bzw. Trocknungsschritt in einer von einem Kneter verschiedenen Vorrichtung, vorzugsweise einem Bandtrockner, erfolgt. Zwischen den beiden Schritten kann noch ein Zerkleinerungsschritt wie ein Hack- oder Wölfschritt vorgesehen sein, um die Oberfläche des Trocken- bzw. Vernetzungsguts zu erhöhen.

Es ist ferner bevorzugt, dass zwischen der Temperatur beim in Kontakt bringen zum Homogenisieren und dem Trocknen bzw. Vernetzen eine Temperaturdifferenz besteht. Die beiden Temperaturen unterscheiden sich um mindestens 10°C, vorzugsweise um mindestens 20°C und besonders bevorzugt um mindestens 40°C sowie darüber hinaus bevorzugt um mindestens 80°C. In einer Ausgestaltung des erfindungsgemäßen Verfahrens liegt die Temperatur während des in Kontakt bringens zum Homogenisieren im Bereich von 2 bis 40°C, vorzugsweise im Bereich von 10 bis 35°C und besonders bevorzugt im Bereich von 15 bis 30°C. Zum Einstellen der geeigneten Temperaturen ist es bevorzugt, dass der Kneter temperierbar ist. Es können entweder das die Knetwelle(n) umgebende Gehäuse oder auch die Knetwellen, gegebenenfalls mit den darauf angeordneten Elementen selbst, oder beide temperierbar sein.

In dem erfindungsgemäßen Verfahren können durch den Kneter eine Kneterenergie im Bereich von 0,01 bis 1 MJ/kg, vorzugsweise im Bereich von 0,25 bis 0,75 MJ/kg und darüber hinaus bevorzugt 0,3 bis 0,7 MJ/kg sowie ein spezifisches Drehmoment von 0,1 bis 70 Nm/l, vorzugsweise im Bereich von 5 bis 50 Nm/l und besonders bevorzugt im Bereich von 10 bis 40 MJ/kg angewandt werden. Geeignete Kneter sind unter anderem in DE 195 36 944 A1, US 5,147,135 und DE 195 33 693 A1 beschrieben. Zudem können geeignete Kneter beispielsweise bei der List AG, Arisdorf, Schweiz kommerziell erhalten werden.

In dem erfindungsgemäßen Verfahren ist das Polysaccharid ein nicht vernetztes Polysaccharid. Das Vernetzungsmittel ist Polyphosphat oder Polyphosphorsäure. Es ist weiterhin erfindungsgemäß bevorzugt Polyphosphat oder Polyphosphorsäure mit anderen geeigneten weiteren Vernetzungsmitteln zu kombinieren. Geeignete weitere Vernetzungsmittel sind beispielsweise Aluminiumchlorid oder Zitronensäure, wie in WO 02/096953 A1 beschrieben, oder Polyamine, wie in US 6,734,298 B1 beschrieben.

Durch die Verwendung von Polyphosphaten oder Polyphosphorsäure als Vernetzungsmittel für Polysaccharide gemäß dem erfindungsgemäßen Verfahren sind wasserabsorbierende Polysaccharide erhältlich, die sich durch ein hervorragendes Absorptions- und Retentionsvermögen für Wasser, wässrige Lösungen und Körperflüssigkeiten auszeichnen. Darüber hinaus ist das durch das erfindungsgemäße Verfahren erhältliche wasserabsorbierende Polysaccharid lagerstabil, im wesentlichen frei von Restmonomerenanteilen und organischen Lösungsmitteln, nur in geringem Maße in wässrigen Flüssigkeiten löslich und in hohem Maße biologisch abbaubar.

Die im erfindungsgemäßen Verfahren eingesetzten Polysaccharide sind wasserlöslich bzw. wasserquellbar und werden in nicht vernetzter Form eingesetzt. Sie können neben den Hydroxylgruppen mit weiteren Gruppen modifiziert sein, insbesondere mit solchen Gruppen, die die Wasserlöslichkeit verbessern. Zu solchen Gruppen gehören beispielsweise die Carboxyl-Gruppe, die Carboxylalkyl-Gruppe, besonders bevorzugt die Carboxymethylgruppe, die Hydroxyalkyl-Gruppe, insbesondere die Hydroxymethylgruppe und/oder die Hydroxyethylgruppe, wobei die Hydroxymethylgruppe besonders bevorzugt ist, sowie die Phosphat-Gruppe.

Je nach funktioneller Modifizierung können die in dem erfindungsgemäßen Verfahren eingesetzten Polysaccharide demnach auf elektrisch geladenen oder auf elektrisch ungeladenen Polysacchariden basieren. Denkbar ist auch ein Einsatz einer auf elektrisch geladenen und elektrisch ungeladenen Polysacchariden basierenden Polysaccharid-Mischung.

Zu den erfindungsgemäß bevorzugten elektrisch ungeladenen Polysacchariden gehören Stärke oder Stärkederivate wie beispielsweise Hydroxypropylstärke, Amylose, Amylopektin, Cellulose oder Cellulosederivate wie beispielsweise Ethylhydroxyethylcellulose oder Hydroxypropylcellulose oder Polygalaktomannane wie beispielsweise Guar oder Johannisbrotkernmehl.

Zu den erfindungsgemäß bevorzugten elektrisch geladenen Polysacchariden gehören insbesondere Polycarboxypolysaccharide. Die in dem erfindungsgemäßen Verfahren vorzugsweise eingesetzten Polycarboxypolysaccharide leiten sich entweder von Polysacchariden ab, die von Natur aus keine Carboxylgruppen enthalten und durch nachträgliche Modifizierung mit Carboxylgruppen versehen werden oder sie enthalten von Natur aus bereits Carboxylgruppen und werden gegebenenfalls nachträglich durch Modifizierung mit weiteren Carboxylgruppen versehen. Zur ersten Gruppe von Polysacchariden zählen beispielsweise oxidierte Stärke, carboxylierte Phosphatstärke, oxidierte Cellulose, Carboxymethylcellulose oder Carboxymethylstärke, wobei von diesen die Carboxymethylcellulose (CMC) besonders bevorzugt ist. Zu den bevorzugten Polysacchariden, die von Natur aus bereits Carboxylgruppen enthalten, gehören beispielsweise, Xanthan, Alginate oder Gummi Arabicum.

Erfindungsgemäß besonders bevorzugt werden Polycarboxypolysaccharide wie beispielsweise Carboxymethylguar, carboxylierte Hydroxyethyl- oder Hydroxypropylcellulose, Carboxymethylcellulose und Carboxymethylstärke, oxidierte Stärke, Xanthan und Mischungen aus den einzelnen Polycarboxypolysacchariden, als Polysaccharide eingesetzt, wobei der Einsatz von Carboxymethylcellulose am meisten bevorzugt ist. Grundsätzlich sind im erfindungsgemäßen Verfahren Polycarboxypolysaccharidderivate mit niedrigen und hohen Carboxyl-Substitutionsgraden einsetzbar. In einer bevorzugten Ausführungsform weisen sie einen durchschnittlichen Carboxyl-Substitutionsgrad im Bereich von 0,3 bis 1,5 auf, insbesondere bevorzugt werden Polycarboxypolysaccharidderivate mit einem Substitutionsgrad im Bereich von 0,4 bis 1,2 im erfindungsgemäßen Verfahren eingesetzt.

In einer bevorzugten Ausführungsform des erfindungsgemäßen Verfahrens werden die Polycarboxypolysaccharide mit einem Zusatz carboxlgruppenfreier Polysaccharide eingesetzt. Bevorzugt werden stark quellende Polysaccharide, wie z.B. Polygalaktomannane oder Hydroxyalkylcellulosen verwendet. Die zur Modifizierung einzusetzenden Mengen carboxlgruppenfreier Polysaccharide werden durch das geforderte Eigenschaftsprofil bestimmt, bevorzugt werden 20 Gew. %, vorzugsweise 10 Gew. % und besonders bevorzugt 5 Gew. % bezogen auf unvernetzte Polycarboxypolysaccharide verwendet.

Die carboxylgruppenfreien Polysaccharide können dabei vor dem in Kontakt bringen mit dem Polyphosphat oder der Polyphosphorsäure mit dem unvernetzten Polycarboxypolysaccharid vermischt werden oder erst nach dem in Kontakt bringen des unvernetzten Polycarboxypolysaccharids mit dem Polyphosphat oder der Polyphosphorsäure mit dem Polycarboxypolysaccharid vermischt werden. Denkbar ist auch, dass die carboxylgruppenfreien Polysaccharide zunächst mit dem Polyphosphat oder der Polyphosphorsäure oder aber mit einer wässrigen Lösung beinhaltend das Polyphosphat oder die Polyphosphorsäure in Kontakt gebracht werden und die so erhaltene Mischung dann mit dem Polycarboxypolysaccharid vermischt wird.

Die Carboxylgruppen der im erfindungsgemäßen Verfahren vorzugsweise eingesetzten unvernetzten Polycarboxypolysaccharide sind mindestens zu 50%, bevorzugt zu mindestens 80%, besonders bevorzugt zu mindestens 90% und ganz besonders bevorzugt zu 100% neutralisiert. Als Neutralisationsmittel haben sich Alkalihydroxide wie Natrium- und Kaliumhydroxid, Natrium- und Kaliumcarbonate bzw. Hydrogencarbonate und Ammoniumhydroxid und Amine bewährt.

Die bevorzugten, im erfindungsgemäßen Verfahren eingesetzten wasserlöslichen Polysaccharide haben im Rahmen der von dem natürlichen Polymeraufbau vorgegebenen Molekulargewichtsverteilung ein hohes mittleres Molekulargewicht und damit auch eine hohe Lösungsviskosität in verdünnter wässriger Lösung wie z.B. aus Baumwoll-Linters hergestellte Carboxymethylcellulose. Bevorzugt sind Polysaccharide mit einer Lösungsviskosität in 1%iger wässriger Lösung von mehr als 2.000 mPas. Wird im erfindungsgemäßen Verfahren ein Polycarboxypolysaccharid eingesetzt, so sollte dieses eine Lösungsviskosität in 1%iger wässriger Lösung von mehr als 5.000 mPas und besonders bevorzugt von mehr als 7.000 mPas aufweisen.

Bedingt durch den Herstellungsprozess können Polysaccharide als Nebenbestandteil unterschiedlich hohe Salzmengen enthalten. Typische Salzgehalte von erfindungsgemäß als Polysaccharide bevorzugten Carboxymethylcellulosen in Lebensmittelqualitäten liegen bei etwa 0,5 Gew.%, bei technischen Qualitäten im Bereich von etwa 2 Gew.% bis hin zu 25 bis 50 Gew.% für Produkte in der Anwendung als Schutzkolloide. Obwohl die durch das erfindungsgemäße Verfahren erhaltenen wasserabsorbierenden Polysaccharide eine hohe Toleranz gegenüber einer Salzfracht aufweisen, sollten die zu verwendenden unvernetzten Polysaccharide einen Salzgehalt von nicht mehr als 20 Gew.%, bevorzugt von nicht mehr als 15 Gew.%, besonders bevorzugt von nicht mehr als 5 Gew. % und darüber hinaus bevorzugt von nicht mehr als 2 Gew.-% Salz, jeweils bezogen auf das Gewicht des im erfindungsgemäßen Verfahren eingesetzten unvernetzten Polysaccharids, aufweisen.

Die physikalische Form der im erfindungsgemäßen Verfahren eingesetzten Polysaccharide ist für die Eigenschaften der durch das erfindungsgemäße Verfahren erhältlichen wasserabsorbierenden Polysaccharide ohne Bedeutung. Daher können die Polysaccharide z.B. in Form von Pulvern, Feinstpulvern, Granulaten, Fasern, Flakes, Perlen oder Kompaktaten eingesetzt verwendet werden, wobei die Verwendung von pulverförmigen Materialien mit einer Korngröße im Bereich von 1 bis 2.000 µm aufgrund der einfachen Dosier- und Förderbarkeit bevorzugt wird.

Als Polyphosphat oder Polyphosphorsäure werden vorzugsweise kettenförmige Polyphosphate (*catena*-Phosphate) oder die ringförmigen Polyphosphate (Cyclophosphate, auch als "Metaphosphate" bezeichnet) eingesetzt, wobei es sich bei den Polyphosphaten um die Salze und die Ester von Polyphosphorsäuren handelt.

Besonders bevorzugte Polyphosphate sind Verbindungen der Zusammensetzung M^{I}ₙ₊₂[PₙO₃ₙ₊₁] oder M^{I}_{n[}H₂PₙO₃ₙ₊₁], wobei Verbindungen der Struktur M^{I}ₙ[H₂PₙO₃ₙ₊₁] besonders bevorzugt sind. Unter diesen sind besonders bevorzugt Verbindungen der Zusammensetzung NaₙH₂PnO₃ₙ₊₁, wie beispielsweise das "*Grahamsche* Salz", das "*Maddrellsche* Salz", das "*Kurrolsche* Salz" oder das in Waschmittteln eingesetzte "*Calgon*"*.*

Bevorzugte Metaphosphate sind Verbindungen der Zusammensetzung M^{I}ₙ[PO₃]ₙ.

In den vorstehend genannten Formeln steht M^{I} für einwertiges Metall, vorzugsweise für Natrium oder Kalium. n weist vorzugsweise einen Wert von mindestens 2, vorzugsweise mindestens 10 und darüber hinaus bevorzugt einen Wert von mindestens 50 auf, wobei ein Wert von 5.000, vorzugsweise von 1.000 und besonders bevorzugt von 100 nicht überschritten wird.

In einer besonderen Ausführungsform des erfindungsgemäßen Verfahrens werden Polyphosphate eingesetzt, die durch Kondensation von Dihydrogenmonophosphaten hergestellt worden sind und in denen die H-Atome der als Kettenendgruppe gebundenen sauren OH-Gruppe nicht durch Metall ersetzt sind. Die besonders bevorzugten Polyphosphate besitzen die Zusammensetzung M^{I}ₙ[H₂PₙO₃ₙ₊₁], wobei M^{I} und n die vorstehend erläuterte Bedeutung besitzen.

Bevorzugte Polyphosphorsäuren sind Polyphosphorsäuren, die durch die kontrollierte Zugabe von Wasser zu P₄O₁₀ oder durch Kondensation beim Erhitzen von H₃PO₄ erhalten werden. Die erfindungsgemäßen bevorzugten Polyphosphorsäuren haben die Zusammensetzung

Hₙ₊₂PₙO₃ₙ₊₁ oder (HPO₃)ₙ, wobei Polyphosphorsäuren der Zusammensetzung (HPO₃)ₙ auch als Metaphosphorsäuren bezeichnet werden, wobei n vorzugsweise einen Wert von mindestens 2, besonders bevorzugt mindestens 10, darüber hinaus bevorzugt mindestens 20 und darüber hinaus besonders bevorzugt mindestens 50 aufweist, wobei vorzugsweise ein Wert von 10.000, besonders bevorzugt von 1.000 und darüber hinaus bevorzugt von 100 nicht überschritten wird.

Mit zunehmendem Wert für n nähert sich die vorstehend genannte Zusammensetzung der Hₙ₊₂PₙO₃ₙ₊₁ der Zusammensetzung (HPO₃)ₙ der Metaphosphorsäuren.

Weiterhin ist es erfindungsgemäß bevorzugt, dass das Polyphosphat oder die Polyphosphorsäure mit dem unvernetzten Polysaccharid in einer Menge in einem Bereich von 0,001 bis 20 Gew.-%, bevorzugt in einer Menge in einem Bereich von 0,01 bis 10 Gew.-% und besonders bevorzugt in einer Menge in einem Bereich von 0,05 bis 5 Gew.-%, jeweils bezogen auf das Gewicht des unvernetzten Polysaccharids, mit dem unvernetzten Polysaccharid in Kontakt gebracht wird.

Es ist auch bevorzugt, dass das Polyphosphat oder die Polyphosphorsäure mit dem unvernetzten Polysaccharid in Gegenwart von Wasser bei einer Temperatur in einem Bereich von 15 bis 60°C, besonders bevorzugt in einem Bereich von 18 bis 40°C und darüber hinaus bevorzugt in einem Bereich von 20 bis 30°C in Kontakt gebracht wird. Am meisten bevorzugt erfolgt das in Kontakt bringen des Polyphosphats oder der Polyphosphorsäure mit dem Polysaccharid bei Raumtemperatur.

Die vorstehend genannten Polyphosphate oder Polyphosphorsäure können alleine oder auch in Kombination mit anderen, nicht auf Polyphosphaten oder Polyphosphorsäuren basierenden Vernetzern zur Vernetzung des Polysaccharids eingesetzt werden. Als zusätzliche, nicht auf Polyphosphaten oder Polyphosphorsäuren basierende Vernetzer sind dabei diejenigen Vernetzer bevorzugt, die in der WO 02/096953 A1 als kovalente oder ionische Nachvernetzungsmittel genannt werden, sowie diejenigen Vernetzer, die in der WO 00/21581 A1 auf der Seite 6 im ersten Absatz genannt werden. Das Gewichtsverhältnis zwischen diesen anderen, nicht auf Polyphosphaten oder Polyphosphorsäuren basierenden Vernetzern und den Polyphosphaten oder Polyphosphorsäuren liegt vorzugsweise in einem Bereich von 1 : 0,01 bis 1 : 50, besonders bevorzugt in einem Bereich von 1 : 0,1 bis 1 : 20 und darüber hinaus bevorzugt in einem Bereich von 1 : 1 bis 1 : 10.

Die Quellzeit ist abhängig von der Temperatur, bei der das Polyphosphat oder die Polyphosphorsäure mit dem unvernetzten Polysaccharid in Kontakt gebracht werden sowie von den eingesetzten Ausgangsverbindungen und kann durch einfache Vorversuche leicht bestimmt werden. Die erste Verfahrensstufe des erfindungsgemäßen Verfahrens ist vorzugsweise dann beendet, wenn eine weitere Volumenzunahme des Polysaccharids in Folge des Anquellens nicht mehr beobachtet werden kann. Vorzugsweise erfolgt das in Kontakt bringen des Polyphosphats oder der Polyphosphorsäure mit dem unvernetzten Polysaccharid für einen Zeitraum von 1 Minute bis 48 Stunden, besonders bevorzugt von 1 Stunde bis 24 Stunden und darüber hinaus bevorzugt von 12 bis 20 Stunden.

Vorzugsweise erfolgt das in Kontakt bringen des unvernetzten Polysaccharids mit dem Polyphosphat oder mit der Polyphosphorsäure bei einem pH-Wert in einem Bereich von 7 bis 13, besonders bevorzugt in einem Bereich von 7,5 bis 12,5 und darüber hinaus bevorzugt in einem Bereich von 8 bis 12. Dieses gilt insbesondere dann, wenn als Polysaccharid ein Polycarboxypolysaccharid eingesetzt wird. Durch die Einstellung des pH-Wertes innerhalb der vorstehend angegebenen pH-Bereiche kommt es dann zu einer zumindest teilweisen Neutralisation der in dem Polysaccharid vorhandenen Carboxylgruppen. Außerdem wird die Polyphosphorsäure ebenso mindestens teilweise neutralisiert.

In einer besonders bevorzugten Ausführungsform des erfindungsgemäßen Verfahrens erfolgt das in Kontakt bringen des unvernetzten Polysaccharids mit dem Polyphosphat oder der Polyphosphorsäure derart, dass zunächst das Polyphosphat oder die Polyphosphorsäure in Wasser gelöst oder dispergiert wird, in der wässrigen Lösung oder der wässrigen Dispersion des Polyphosphats oder der Polyphosphorsäure ein pH-Wert in einem Bereich von 7 bis 13, vorzugsweise von 7,5 bis 12,5 und besonders bevorzugt von 8 bis 12 eingestellt wird und anschließend die wässrige Lösung oder die wässrige Dispersion des Polyphosphats oder der Polyphosphorsäure mit einem unvernetzten Polysaccharid in Kontakt gebracht wird.

In einer anderen besonderen Ausführungsform des erfindungsgemäßen Verfahrens erfolgt das in Kontakt bringen des unvernetzten Polysaccharids mit dem Polyphosphat oder der Polyphosphorsäure derart, dass das unvernetzte Polysaccharid zunächst mit dem Polyphosphat oder der Polyphosphorsäure unter trockenen Bedingungen vermischt wird und die so erhaltene Mischung anschließend mit Wasser in Kontakt gebracht wird. Dabei wird vorzugsweise durch Zugabe von Säuren oder Basen zum Wasser oder zur Mischung aus dem Polycarboxypolysaccharid und dem Polyphosphat oder der Polyphosphorsäure sichergestellt, dass das in Kontakt bringen des unvernetzten Polysaccharids mit dem Polyphosphat oder der Polyphosphorsäure bei einem pH-Wert in einem Bereich von 7 bis 13, vorzugsweise von 7,5 bis 12,5 und besonders bevorzugt von 8 bis 12 erfolgt.

In einer weiteren besondern Ausführungsform des erfindungsgemäßen Verfahrens erfolgt das in Kontakt bringen des unvernetzten Polysaccharids mit dem Polyphosphat oder der Polyphosphorsäure derart, dass zunächst das unvernetzte Polysaccharid mit Wasser in Kontakt gebracht wird und anschließend das gequollene Polysaccharid mit dem Polyphosphat oder der Polyphosphorsäure in Kontakt gebracht wird. Auch hierbei wird vorzugsweise durch Zugabe von Säuren oder Basen zum Wasser oder zum mit dem Wasser in Kontakt gebrachten Polysaccharid oder zum Polyphosphat bzw. zur Polyphosphorsäure sichergestellt, dass das in Kontakt bringen des unvernetzten Polysaccharids mit dem Polyphosphat oder der Polyphosphorsäure bei einem pH-Wert in einem Bereich von 7 bis 13, vorzugsweise von 7,5 bis 12,5 und besonders bevorzugt von 8 bis 12 erfolgt.

Es ist weiterhin erfindungsgemäß bevorzugt, dass das in Kontakt bringen des unvernetzten Polysaccharids mit dem Polyphosphat oder der Polyphosphorsäure in Gegenwart eines Hilfsmittels erfolgt, wobei das Hilfsmittel zuvor dem unvernetzten Polysaccharid oder mit dem Polyphosphat oder der Polyphosphorsäure vermischt werden kann oder dem bereits mit dem Polyphosphat oder der Polyphosphorsäure in Kontakt gebrachten unvernetzten Polysaccharid zugesetzt werden. Erfolgt das in Kontakt bringen des unvernetzten Polysaccharids mit dem Polyphosphat oder der Polyphosphorsäure derart, dass zunächst eine wässrige Lösung oder eine wässrige Dispersion des Polyphosphats oder der Polyphosphorsäure hergestellt wird, der dann das Polysaccharid zugesetzt wird, so kann das Hilfsmittel auch der wässrigen Lösung oder der wässrigen Dispersion des Polyphosphats oder der Polyphosphorsäure zugesetzt werden.

Die Hilfsmittel können dabei in einer Menge in einem Bereich von 0,01 bis 20 Gew.-%, bevorzugt in einer Menge in einem Bereich von 0,1 bis 10 Gew.-% und besonders bevorzugt in einer Menge in einem Bereich von 1 bis 5 Gew.-%, jeweils bezogen auf das Gewicht des unvernetzten Polysaccharids, zugesetzt werden.

Bevorzugte Hilfsmittel sind Antiblockingadditive, die die Verarbeitbarkeit des entstehenden Hydrogels verbessern und die zumindest teilweise nach der Trocknung in dem Produkt verbleiben. Bevorzugte Antiblockingadditive sind native oder synthetische Fasermaterialien oder andere Materialien mit einer großen Oberfläche z.B. aus der Gruppe der Kieselgele und synthetischen Kieselsäuren und der wasserunlöslichen Mineralsalze.

Weitere bevorzugte Hilfsmittel sind wasserlösliche Hilfsmittel aus der Gruppe der Basen, Salze und Treibmittel. Als Treibmittel werden anorganische oder organische Verbindungen gewählt, die unter Einfluss von Katalysatoren oder Wärme Gas freisetzen, beispielsweise Azo- und Diazoverbindungen, Carbonatsalze, Ammoniumsalze oder Harnstoff.

Weitere Hilfsmittel sind pH-Regulatoren wie z.B. Alkalimetallhydroxide, Ammoniak, basische Salze wie z.B. Alkalimetallcarbonate oder -Acetate. Weitere Hilfsmittel sind Neutralsalze, wie z.B. Alkalimetall- oder Erdalkalimetallsulfate oder - chloride zur Regulierung der Ionenstärke der Lösung bzw. des Salzgehaltes des pulverförmigen Absorberharzes.

Ferner können in dem wässrigen Hydrogel wassermischbare, organische Lösemittel, bevorzugt unter 100°C siedend, als Hilfsmittel eingesetzt werden. Im Zuge der nachfolgenden Trocknung entweichen diese flüchtigen organischen Lösemittel weitestgehend aus dem Hydrogel. Bei der anschließenden Oberflächennachvernetzung werden diese Lösemittel dann endgültig verflüchtigt.

Das in Kontakt bringen des unvernetzten Polysaccharids mit dem Polyphosphat oder der Polyphosphorsäure in Gegenwart von Wasser kann kontinuierlich oder diskontinuierlich, vorzugsweise kontinuierlich, erfolgen. Geeignete Mischeinrichtungen stellen z.B. diskontinuierliche Kneter wie Trogkneter, Innenmischer oder kontinuierliche Kneter wie Ein-, Zwei- oder Mehrwellenmischer dar.

Bei der Herstellung des Polysaccharid-Gels in der ersten Verfahrensstufe des erfindungsgemäßen Verfahrens kann der Gehalt an Polysaccharid in der Mischung aus Polysaccharid, Wasser und Polyphosphat oder Polyphosphorsäure in weiten Grenzen schwanken, in einer bevorzugten Ausführungsform des Verfahrens liegt er im Bereich von 5 bis 65 Gew. %, besonders bevorzugt 10 bis 50 Gew. % und darüber hinaus bevorzugt 15 bis 30 Gew.-%.

In einer bevorzugten Ausführungsform wird das Wasser bzw. die wässrige Lösung oder wässrige Dispersion des Polyphosphats oder der Polyphosphorsäure dem trockenen Rohstoff Polysaccharid kontinuierlich zugeführt, beispielsweise in einem Extruder, wobei das Verfahren so geführt wird, dass das Wasser im Unterschuss vorliegt.

Die Mischung aus Polysaccharid, Polyphosphat oder Polyphosphorsäure und Wasser kann erfindungsgemäß zusätzlich bis zu 30 Gew. %, vorzugsweise bis zu 20 Gew. % eines oder mehrerer, mit Wasser mischbarer und mit dem Polysaccharid nicht mischbarer organischer Lösemittel enthalten.

Vorzugsweise erfolgt das in Kontakt bringen des unvernetzten Polysaccharids mit dem Polyphosphat oder mit der Polyphosphorsäure jedoch in Abwesenheit eines organischen Lösungsmittels.

Als besonders günstig hat sich erwiesen, wenn das gequollene Gel vor dem Vernetzen zerkleinert wird. Durch die Gelzerkleinerung wird vor allem das Verhältnis von Geloberfläche zu Gelvolumen vergrößert, wodurch der nachfolgende Trocknungsschritt wesentlich weniger Energieeintrag benötigt. Das Verfahren der Gelzerkleinerung unterliegt keiner Einschränkung. In einer besonders bevorzugten Ausführungsweise erfolgt die Gelzerkleinerung durch die Verpressung des Gels durch eine Lochscheibe zu Gelsträngen, die gegebenenfalls durch ein Schneidwerkzeug in kürzere Gelstränge zerteilt werden können.

Die Gelkonsistenz kann über die Art und die Menge der Zugabe von Polyphosphaten oder Polyphosphorsäure gezielt eingestellt werden. Eine diesbezügliche Verwendung organischer Lösungsmittel, wie in WO 02/096953 A1 beschrieben, ist hierzu überraschenderweise nicht erforderlich.

In der zweiten Stufe des erfindungsgemäßen Verfahrens wird das zerkleinerte Polysaccharid-Gel unter Bildung eines vernetzten Polysaccharids vernetzt und vorzugsweise gleichzeitig bis auf einen geringen Restwassergehalt getrocknet. Denkbar ist auch, das Polysaccharid-Gel zunächst unter Bedingungen, die nicht zu einem Trocknen des Polysaccharid-Gels führen, zu vernetzen und erst anschließend das vernetzte Polysaccharid-Gel zu trocknen.

Der Vernetzungsschritt kann sich dabei unmittelbar an die Vorquellung anschließen, es ist aber auch möglich, die zerkleinerten Polysaccharid-Gele vor der Weiterverarbeitung für einen längeren Zeitraum, z.B. mehrere Wochen zwischenzulagern, ohne dass sich die Eigenschaften der daraus resultierenden erfindungsgemäßen Superabsorber ändern.

Vorzugsweise wird das Polysaccharid-Gel bei einer Temperatur oberhalb von 70°C, bevorzugt oberhalb von 100°C und besonders bevorzugt oberhalb von 115°C vernetzt und dabei vorzugsweise gleichzeitig getrocknet, wobei vorzugsweise eine Vernetzungs- bzw. Trocknungstemperatur von 300°C, besonders bevorzugt von 250°C und darüber hinaus bevorzugt von 200°C nicht überschritten wird. Denkbar ist auch, das Polysaccharid-Gel zunächst bei geringeren Temperaturen als 70°C, vorzugsweise unter reduziertem Druck, zu trocknen, und erst anschließend durch Erhöhung des getrockneten Polysaccharids auf eine Temperatur, die eine Vernetzung des Polysacharides ermöglicht, zu erhitzen. Grundsätzlich kann der Vernetzungsschritt bei jeder denkbaren Temperatur durchgeführt werden, sofern die Temperatur hoch genug ist, um eine zumindest teilweise Vernetzung des Polysaccharid-Gels durch das Polyphosphat oder die Polyphosphorsäure zu ermöglichen und eine Temperatur nicht übersteigt, die zu einer Zersetzung des Polysaccharids führt.

Bei den Vernetzungs- bzw. Trocknungstemperaturen ist zu beachten, dass sich die Parameter wie der Polymergehalt des Gels, der pH-Wert der Mischung, das Mischverfahren, die Vernetzungs- bzw. Trocknungstemperatur und die Trocknungsdauer gegenseitig beeinflussen und bevorzugt so aufeinander abgestimmt werden, dass während der Vernetzung des Polysacharides mit dem Polyphosphat oder der Polyphosphorsäure keine interne Vernetzung des Hydrogels erfolgt. Wird z.B. bei der Herstellung des Polysaccharid-Gels eine wässrige Lösung mit einem pH-Wert unterhalb von 7 verwendet, wird bei einem Einsatz von Polycarboxypolysacchariden ein Teil der im Polysaccharidderivat vorhandenen Carboxylatgruppen in die freie Säureform überführt, welche vor allem gegen Ende der Trocknung durch eine Veresterung mit den Hydroxylgruppen als interne Vernetzer fungieren können. Um diese, im Prinzip unerwünschte, interne Vernetzung zu vermeiden oder weitgehend zurückzudrängen erfolgt die Vernetzung bzw. Trocknung in diesen Fällen bevorzugt bei Temperaturen im Bereich von 70-100°C. Der pH-Wert wird für gewöhnlich auf 6 oder höher eingestellt. In einer bevorzugten Ausführung der Erfindung wird für die Herstellung des Polysaccharid-Gels eine wässrige Lösung mit einem pH-Wert von ≥ 7 gewählt und die Vernetzung bzw. Trocknung bei Temperaturen ab 110°C, vorzugsweise ab 115 bis 120°C durchgeführt.

Zur Trocknung der Polysaccharid-Gele sind verschiedene Verfahren bekannt. Mögliche Verfahren sind z.B. die Verdampfungstrocknung, Verdunstungstrocknung, Strahlungstrocknung (Beispiel: Infrarottrocknung), Hochfrequenztrocknung (Beisp.: Mikrowellentrocknung), Vakuumtrocknung, Gefriertrocknung oder Sprühtrocknung. So kann die Trocknung beispielsweise nach dem Dünnfilm-Trockenverfahren, z.B. mit Hilfe eines Zweiachsen-Walzentrockners, nach dem Plattentrockenverfahren, gemäß dem die Hydrogelpolymerteilchen auf Platten in mehreren Schichten in eine Trockenkammer geladen werden, in der Heißluft zirkuliert, nach dem Drehtrommel-Verfahren mit Hilfe von Walzentrocknern oder nach dem Förderband-Verfahren, im folgenden auch als Bandtrocknung bezeichnet erfolgen. Die Bandtrocknung, bei der mit Löcher versehene Horden eines Kreisförderers in einem Tunnel mit Trocknungsgut beladen und das Trocknungsgut während der Förderung durch Durchblasen von Heißluft durch die Hordenlöcher getrocknet wird, stellt das wirtschaftlichste Trocknungsverfahren für wasserquellbare hydrophile Hydrogele dar und ist daher bevorzugt.

Die Feuchtigkeit des durch Trocknung des Polysaccharid-Gels entstandenen Polymers liegt vorteilhafter Weise nicht über 30 Gew. %, vorzugsweise nicht über 15 Gew. % und besonders bevorzugt nicht über 10 Gew. %.

Wird das Polysaccharid-Gel in einem kontinuierlichen Mischer hergestellt, beispielsweise in einem Extruder, so können die noch nicht an der Oberfläche nachvernetzten Vorprodukte bereits ab pH-Werten von 7 hohe Retentionen von größer oder gleich 40 g/g aufweisen, die sich beim Tempern über 60 Minuten und 120°C als stabil erweisen und die sich von Produkten, die mit höheren pH-Werten hergestellt wurden, nur noch geringfügig unterscheiden. Werden die Hydrogele dagegen in einem Batch-Prozess hergestellt, so steigt die Stabilität gegenüber einer Temperung mit steigendem pH-Wert des Gels an. Eine bevorzugte pH-Einstellung bei der Hydrogelbildung im Batch-Prozess liegt daher bei pH 10 oder höher.

In dem erfindungsgemäßen Verfahren wird in einem zusätzlichen Verfahrensschritt das nach dem Trocknen des zerkleinerten Polysaccharid-Gels erhaltene vernetzte Polycarboxypolysaccharid in einem weiteren Verfahrensschritt gemahlen. Durch die Zerkleinerung des Polysaccharid-Gels sowie durch das Mahlen des getrockneten, vernetzten Polycarboxypolysaccharids werden so partikuläre, vernetzte Polysaccharide erhalten.

Für die nachfolgende Mahlung der getrockneten und zuvor zerkleinerten Polysaccharid-Gele ist es vorteilhaft, das Trockengut im letzten Abschnitt der bevorzugten Bandtrocknung auf Temperaturen <70°C, bevorzugt <60°C und besonders bevorzugt <50°C abzukühlen. Die getrockneten, abgekühlten Polysaccharid-Gele bzw. zerkleinerten Polysaccharid-Gele werden zunächst vorgebrochen, beispielsweise mit Hilfe eines Fingerbrechers. Die so vorzerkleinerten getrockneten Gelteilchen werden dann gemahlen, wobei die Mahlung bevorzugt mit Hilfe eines Walzenstuhls erfolgt, um den Anfall an Feinteilen möglichst gering zu halten. In einer besonders bevorzugten Ausführung erfolgt die Mahlung zweistufig, erst über einen Grobwalzenstuhl, dann über einen Feinwalzenstuhl, wobei letzterer wiederum ein- oder zweistufig sein kann.

Bei der anschließenden Siebung wird die Korngrößenverteilung eingestellt, die in der Regel zwischen 10 und 3000 µm, bevorzugt zwischen 100 und 2000 µm und besonders bevorzugt zwischen 150 und 850 µm liegt. Zu grobe Partikel können erneut der Mahlung unterworfen werden, zu feinteilige Partikel können dem Herstellungsprozess zurückgeführt werden.

In dem erfindungsgemäßen Verfahren schließt sich an den Trocknungsschritt bzw. an den Mahlungsschritt noch ein weiterer Verfahrensschritt an, in dem das partikuläre, vernetzte Polysaccharid im Außenbereich der Partikel mit einem Nachvernetzungsmittel nachvernetzt wird.

Dabei wird als Außenbereich der Partikel vorzugsweise jedes Volumenelement des Partikels verstanden, dessen Abstand zum Mittelpunkt der Partikel mindestens 75%, vorzugsweise mindestens 85% und besonders bevorzugt mindestens 95% des äußeren Radius der Polymerpartikel beträgt.

Die Oberflächenvernetzung des getrockneten, partikulären, vernetzten Polycarboxypolysaccharids erfolgt vorzugsweise mit 0,001 bis 25 Gew. %, besonders bevorzugt mit 0,1 bis 20 Gew. % des Nachvernetzungsmittels, jeweils bezogen auf das Gewicht des vernetzten Polysaccharids. Dabei wird das Nachvernetzungsmittel vorzugsweise in Form einer 0,01 bis 80 Gew.-%igen, vorzugsweise einer 0,1 bis 60 Gew.-%igen Lösung eingesetzt. Das Zuführen des Nachvernetzungsmittels erfolgt dabei in geeigneten Mischaggregaten. Dies sind beispielsweise Paterson-Kelly-Mischer, DRAIS-Turbulenzmischer, Lödigemischer, Rubergmischer, Schneckenmischer, Tellermischer, Wirbelschichtmischer oder Schugi-Mischer. Nach Aufsprühen der Lösung des Nachvernetzungsmittels kann ein Temperaturbehandlungsschritt folgen, bevorzugt in einem nachgeschalteten Trockner, bei einer Temperatur zwischen 40 und 250°C, bevorzugt 60-200°C und besonders bevorzugt 80-160°C, über einen Zeitraum von 5 Minuten bis 6 Stunden, bevorzugt 10 Minuten bis 2 Stunden und besonders bevorzugt 10 Minuten bis 1 Stunde, wobei Lösungsmittelanteile entfernt werden. Die optimale Zeitdauer der Nacherhitzung kann für die einzelnen Vernetzertypen mit wenigen Versuchen leicht ermittelt werden. Sie wird dadurch begrenzt, wenn das gewünschte Eigenschaftsprofil des Superabsorbers infolge von Hitzeschädigung wieder zerstört wird. Die thermische Behandlung kann in üblichen Trocknern oder Öfen durchgeführt werden; beispielhaft seien Drehrohröfen, Wirbelbetttrockner, Tellertrockner, Paddeltrockner oder Infrarottrockner genannt.

Es hat sich teilweise als vorteilhaft erwiesen, dass die wässrige Lösung des Oberflächennachvernetzers vor ihrem Einsatz auf eine Temperatur von 15°C - 100°C, vorzugsweise auf 20°C - 60°C eingestellt wird.

Die kovalente Oberflächennachvernetzung kann gegebenenfalls durch Katalysatoren beschleunigt werden. Als Katalysatoren werden bevorzugt Verbindungen eingesetzt, die die Veresterungsreaktion zwischen einer Carboxylgruppe und einer Hydroxylgruppe katalysieren wie z.B. Hypophosphite, Acetylacetonate, Mineralsäuren, wie z.B. Schwefelsäure und Lewis Säuren. Bevorzugt werden Schwefelsäure und Hypophosphit verwendet. Das Gewichtsverhältnis von Oberflächennachvernetzer zu Vernetzungskatalysator beträgt 1:0,001 - 1:1, vorzugsweise 1:0,1 - 2:1

In einer bevorzugten Ausführungsform werden die Vernetzungskatalysatoren der Lösung des Oberflächennachvernetzers zugemischt.

Optional kann die Nachvernetzungslösung bis zu 70 Gew.% eines oder mehrerer Hilfsmittel enthalten. Hilfsmittel sind vor allem wasserlösliche Verbindungen, die die homogene Verteilung der Vernetzerlösung auf der Oberfläche der Absorber fördern, indem sie die Penetration des Lösungsmittels in das Innere der Superabsorberpartikel verlangsamen sowie die Löslichkeit der Partikeloberfläche und damit die Tendenz der feuchten Superabsorberpartikel miteinander zu verkleben reduzieren. Bevorzugte Hilfsmittel sind neben wassermischbaren organischen Lösungsmitteln wie beispielsweise Ethanol, Propanol, 2-Propanol, Aceton, Glycerin, Tetrahydrofuran und Dioxan auch wasserlösliche hydrophile organische Feststoffe, insbesondere Polymere wie z.B. Polyalkylenglykole, Polyvinylalkohole, bevorzugt Polyäthylenglykole.

Die Nachvernetzung des Außenbereiches kann durch ionische oder kovalente Nachvernetzungsmittel bewerkstelligt werden, die mit den oberflächennahen funktionellen Molekülgruppen, vorzugsweise Carboxyl-, Carboxylat- oder Hydroxylgruppen, vorzugsweise unter Erhitzung reagieren.

Als kovalente Nachvernetzungsmittel, die auch in Kombination mit ionischen Vernetzern eingesetzt werden können, werden solche Vernetzer eingesetzt, die mit den funktionellen Gruppen der Polysaccharide unter Ausbildung kovalenter Bindungen reagieren. In einer bevorzugten Ausführungsform werden Vernetzer eingesetzt, die mit den Hydroxylgruppen, oder bei einem Einsatz von Polycarboxypolysacchariden, mit den Carboxylgruppen des vernetzten Polysaccharids reagieren können, beispielsweise Säuregruppen enthaltende Substanzen. Insbesondere sind niedermolekulare Polycarbonsäuren und deren Derivate wie z.B. Malonsäure, Maleinsäure, Maleinsäureanhydrid, Weinsäure und polymere Polycarbonsäuren, z.B. auf Basis von (Meth)Acrylsäure und/oder Maleinsäure geeignet. Bevorzugt werden Citronensäure, Butantetracarbonsäure und Polyacrylsäure, besonders bevorzugt wird die Citronensäure verwendet. Die Polycarbonsäuren können auch in teilneutralisierter Form, z.B. durch teilweise Neutralisation mit Alkalihydroxiden oder Aminbasen verwendet werden. Neben diesen Nachvernetzungsmitteln sind insbesondere auch Polyphosphate und Polyphosphorsäuren als Nachvernetzungsmittel bevorzugt, wobei vorzugsweise diejenigen Polyphosphate und Polyphosphorsäure eingesetzt werden, die bereits im Zusammenhang mit der ersten Verfahrenstufe des erfindungsgemäßen Verfahrens genannt wurden.

Geeignete ionische Nachvernetzungsmittel, die alleine oder in Kombination mit den kovalenten Nachvernetzungsmitteln verwendet werden können, sind Salze von mindestens zweiwertigen Metall-Kationen, beispielsweise Erdalkaliionen wie Mg²⁺, Ca²⁺, sowie Al³⁺, Ti⁴⁺, Fe²⁺/Fe³⁺, Zn²⁺ oder Zr⁴⁺, wobei Al³⁺, Ti⁴⁺ und Zr⁴⁺ bevorzugt sind und Al³⁺ besonders bevorzugt ist. Aluminiumsalze werden bevorzugt in einer Menge von 0,2 - 1,0 Gew.%, vorzugsweise 0,25 - 0,85 Gew.% bezogen auf das vernetzte Polysaccharid eingesetzt.

Die Salze der Metallkationen können sowohl allein als auch im Gemisch untereinander eingesetzt werden. Die Metallkationen in Form ihrer Salze besitzen eine ausreichende Löslichkeit im verwendeten Lösungsmittel, besonders bevorzugt werden die Metallsalze mit schwach komplexierenden Anionen wie z.B. Chlorid, Nitrat, Sulfat und Acetat eingesetzt.

Weitere geeignete Nachvernetzungsmittel sind solche, die sowohl kovalente als auch ionische Vernetzungsbindungen eingehen können, z.B. Di- und Polyamine die sowohl als kovalente Vernetzer, über Amidgruppen, wie auch als ionische Vernetzer, über Ammoniumsalzkomplexe, fungieren können.

In einer besonders bevorzugten Ausführungsform des erfindungsgemäßen Verfahrens werden Polyphosphate oder Polyphosphorsäuren als Nachvernetzungsmittel eingesetzt, in einer anderen, besonders bevorzugten Ausführungsform kommt eine Mischung aus Polyphosphaten oder Polyphosphorsäuren und mindestens einem weiteren der vorstehend genannten, nicht auf Polyphosphaten oder Polyphosphorsäuren basierenden Nachvernetzungsmittel, insbesondere Mischungen aus Polyphosphaten oder Polyphosphorsäuren und ionischen Nachvernetzungsmitteln, zum Einsatz wobei Mischungen aus Polyphosphaten oder Polyphosphorsäuren und Aluminiumsalzen ganz besonders bevorzugt sind.

Bei der Verwendung von Polyphosphaten oder Polyphosphorsäuren als Nachvernetzungsmittel werden diese vorzugsweise in Form einer wässrigen Lösung mit einem pH-Wert in einem Bereich von 7 bis 13, besonders bevorzugt in einem Bereich von 8 bis 12 eingesetzt. Bei der Verwendung von Polyphosphaten oder Polyphosphorsäuren als Nachvernetzungsmittel ist es weiterhin bevorzugt, dass die Polyphosphate oder die Polyphosphorsäuren in einer Menge in einem Bereich von 0,01 bis 10 Gew.-%, besonders bevorzugt in einer Menge in einem Bereich von 0,1 bis 5 Gew.-% und besonders bevorzugt in einer Menge in einem Bereich von 0,3 bis 1,5 Gew.-%, jeweils bezogen auf das Gewicht der vernetzten Polysaccharide, eingesetzt werden.

Im Zusammenhang mit der Nachvernetzung der vernetzten Polysaccharide ist es in einer besonderen Ausführungsform des erfindungsgemäßen Verfahrens bevorzugt, dass das vernetzte Polysaccharid mit einem anorganischen Material in Kontakt gebracht wird.

Als anorganisches Material kann jedes dem Fachmann bekannte, vorzugsweise partikuläre anorganische Material mit den vernetzten Polysacchariden in Kontakt gebracht werden, das zur Modifizierung der Eigenschaften wasserabsorbierender Polymere geeignet ist. Zu den bevorzugten anorganischen Materialien gehören Silikate, insbesondere Gerüstsilikate wie Zeolithe oder Silikate, die durch Trocknung wässriger Kieselsäurelösungen oder Kieselsolen erhalten wurden, beispielsweise die kommerziell erhältlichen Produkte wie Fällungskieselsäuren und pyrogene Kieselsäuren, beispielsweise Aerosile, Aluminate, Titandioxide, Zinkoxide, Tonmaterialien und weitere dem Fachmann geläufige Mineralien sowie kohlenstoffhaltige anorganische Materialien.

Bevorzugte Silikate sind alle natürlichen oder synthetischen Silikate, die in "Holleman und Wiberg, Lehrbuch der Anorganischen Chemie, Walter de Gruyter-Verlag, 91.-100. Auflage, 1985" auf den Seiten 750 bis 783, als Silicate offenbart sind. Der vorstehend genannte Abschnitt dieses Lehrbuchs wird hiermit als Referenz eingeführt und gilt als Teil der Offenbarung der vorliegenden Erfindung.

Besonders bevorzugte Silikate sind die Zeolithe. Als Zeolithe können alle dem Fachmann bekannten synthetischen oder natürlichen Zeolithe eingesetzt werden. Bevorzugte natürliche Zeolithe sind Zeolithe aus der Natrolith-Gruppe, der Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS.

Als Zeolithe können Zeolithe des sogenannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie β-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Als Aluminate werden vorzugsweise die in der Natur vorkommenden Spinelle, insbesondere gewöhnlicher Spinell, Zinkspinell, Eisenspinell oder Chromspinell eingesetzt.

Bevorzugte Titandioxide sind das Titandioxid in den Kristallformen Rutil, Anatas und Brookit, sowie eisenhaltige Titandioxide wie beispielsweise Ilmenit, calciumhaltige Titandioxide wie Titanit oder Perowskit.

Bevorzugte Tonmaterialien sind diejenigen, die in "Holleman und Wiberg, Lehrbuch der Anorganischen Chemie, Walter de Gruyter-Verlag, 91.-100. Auflage, 1985" auf den Seiten 783 bis 785, als Tonmaterialien offenbart sind. Der vorstehend genannte Abschnitt dieses Lehrbuchs wird hiermit als Referenz eingeführt und gilt als Teil der Offenbarung der vorliegenden Erfindung. Besonders bevorzugte Tonmaterialien sind Kaolinit, Illit, Halloysit, Montmorillonit sowie Talk.

Bevorzugte kohlenstoffhaltige, jedoch nicht organische Materialien sind diejenigen Kohlenstoffe, die in "Holleman und Wiberg, Lehrbuch der Anorganischen Chemie, Walter de Gruyter-Verlag, 91.-100. Auflage, 1985" auf den Seiten 705 bis 708 als Graphite genannt sind. Der vorstehend genannte Abschnitt dieses Lehrbuchs wird hiermit als Referenz eingeführt und gilt als Teil der Offenbarung der vorliegenden Erfindung. Besonders bevorzugte Graphite sind künstliche Graphite wie beispielsweise Koks, Pyrographit, Aktivkohle oder Ruß.

Bei der Verwendung der vorstehend genannten anorganischen Materialien oder deren Mischungen ist es besonders bevorzugt, dass diese Materialien in einer Menge in einem Bereich von 0,1 bis 1 Gew.-%, darüber hinaus bevorzugt in einer Menge in einem Bereich von 0,25 bis 0,75 Gew.-% und darüber hinaus noch mehr bevorzugt in einem Bereich von 0,4 bis 0,6 Gew.-%, bezogen auf das Gesamtgewicht der vernetzten Polysaccharide, mit den vernetzten Polysacchariden in Kontakt gebracht werden.

Es ist erfindungsgemäß weiterhin bevorzugt, dass die anorganischen Materialien eine nach der BET-Methode bestimmte spezifische Oberfläche in einem Bereich von 30 bis 850 m²/g, vorzugsweise in einem Bereich von 40 bis 500 m²/g, besonders bevorzugt in einem Bereich von 100 bis 300 m²/g und darüber hinaus bevorzugt in einem Bereich von 150 bis 250 m²/g aufweisen. Im allgemeinen und in dem Fall, dass es sich bei den anorganischen Materialien um Sipernate oder Aerosile handelt, liegt die Oberfläche in einem Bereich von 30 bis 850 m²/g, vorzugsweise in einem Bereich von 40 bis 500 m²/g, besonders bevorzugt in einem Bereich von 100 bis 300 m²/g und wird mit Stickstoff in einem Areameter nach ISO 5794, Annex D bestimmt.

Bei einem Einsatz anorganischer Materialien in Form von Partikeln ist es weiterhin bevorzugt, dass mindestens 90 Gew.-%, vorzugsweise mindestens 95 Gew.-% und darüber hinaus bevorzugt mindestens 99 Gew.-% des anorganischen Materials eine Partikelgröße von weniger als 200 µm, besonders bevorzugt von weniger als 100 µm und darüber hinaus bevorzugt von weniger als 1 µm und darüber hinaus bevorzugt von weniger als 500 nm und darüber hinaus noch mehr bevorzugt von weniger als 100 nm aufweist. Die Sipernate weisen eine Teilchengröße im Bereich von 10 bis 180 µm, vorzugsweise im Bereich von 20 bis 150 µm und besonders bevorzugt im Bereich von 30 bis 110 µm auf. Die Sipernate weisen in eine anderen Ausgestaltung der vorliegenden Erfindung eine Teilchengröße im Bereich von 1 bis 40 µm, vorzugsweise im Bereich von 2 bis 30 µm und besonders bevorzugt im Bereich von 3 bis 20 µm auf. Hierbei handelt es sich jeweils um die nach der Multisizer-Kapilar-Methode nach ASTM C690-1992 bestimmten mittleren Teilchengröße. Aerosile sind durch eine Teilchengröße im Bereich von 5 bis 50 nm, vorzugsweise im Bereich von 8 bis 20 nm (wie "Aerosil 200" der Degussa AG) gekennzeichnet. Die Teilchengröße lässt sich nach ASTM C 690-1992 mit einem Multisizer bestimmen.

Bei einem Einsatz anorganischer Materialien ist es weiterhin bevorzugt, dass das in Kontakt bringen des vernetzen Polysaccharids mit dem anorganischen Material vorzugsweise in Gegenwart eines "Bindemittels" erfolgt. Dieses wird vorzugsweise zum in Kontakt bringen als Lösung bereitgestellt. Bei dieser Lösung handelt es sich bevorzugt um eine wässrige Lösung. Als Bindemittel kommen alle dem Fachmann geeignet erscheinenden organischen Polymere in Betracht. Besonders bevorzugte Polymere weisen einen Schmelzpunkt nach ISO 11357 im Bereich von -15 bis 150°C, vorzugsweise im Bereich von -12 bis 100°C und besonders bevorzugt im Bereich von - 9 bis 90°C auf. Als Bindemittel sind Polyethylenglykole bevorzugt.

Die Bindemittel liegen vorzugsweise als Film vor. Dieser Film befindet sich bevorzugt auf der Oberfläche des erfindungsgemäßen wasserabsorbierenden Polysaccharids. Dieser Film weist vorzugsweise eine Dicke im Bereich von 0,001 bis 20 nm, vorzugsweise im Bereich von 0,01 bis 15 nm und besonders bevorzugt im Bereich von 0,1 bis 10 nm auf. Die Dicke kann beispielsweise anhand von geeigneter Mikroskope vermessen werden. Hierbei bietet es sich an, einen Durchschnitt von mindestens 10 Schnitten zu bilden. Es ist durchaus möglich, dass der Film nur Teile der Oberfläche des erfindungsgemäßen wasserabsorbierenden Polysaccharids bedeckt.

Als Bindemittel eignen sich in der Regel polymere Materialien mit einem Molekulargewicht von mehr als etwa 290 g/Mol, die eine entsprechende Schmelztemperatur aufweisen und bei einer entsprechenden Anwendungstemperatur keine Zersetzung oder anderweitige für die klebende Wirkung nachteilige Veränderung des Molekülaufbaus zeigen.

Das durch Gelpermeationschromatographie (GPC) bestimmte Zahlenmittel des Molekulargewichts (Mₙ) der als Bindemittel einsetzbaren Polymere liegt vorzugsweise im Bereich von 290 und bis 1.000.000, besonders bevorzugt im Bereich von 1.000 bis 100.000 und darüber hinaus bevorzugt im Bereich von 5.000 bis 20.000 g/Mol.

Die Molekulargewichtsverteilung der genannten Polymere, wie sie ebenfalls durch Gelpermeationschromatographie (GPC) ermittelt werden kann, kann monomodal sein. Gegebenenfalls kann ein als Bindemittel einsetzbares Polymeres auch eine bi- oder höhermodale Verteilung aufweisen.

Weiterhin ist es bei der Verwendung von Bindemitteln bevorzugt, dass diese in einer Menge in einem Bereich von 0,001 bis 10 Gew.-%, vorzugsweise 0,01 bis 5 Gew.-% und darüber hinaus bevorzugt 0,05 bis 2,5 Gew.-%, bezogen auf das Gesamtgewicht des vernetzten Polysaccharids, eingesetzt werden.

Bei einem Einsatz anorganischer Materialien, gegebenenfalls in Kombination mit Bindemitteln, können diese Zusatzkomponenten vor der Nachvernetzung, während der Nachvernetzung oder auch nach der Nachvernetzung der vernetzten Polysaccharide mit den Polysacchariden in Kontakt gebracht werden, wobei der Zusatz dieser Komponenten nach der Nachvernetzung besonders bevorzugt ist. Erfolgt der Zusatz des anorganischen Materials und des Bindemittels vor der Nachvernetzung der vernetzten Polysaccharide, so kann durch Erhitzen des Polysaccharids auf eine Temperatur im Bereich von 100 bis 160 °C und bevorzugt von 120 bis 140 °C zeitgleich die Nachvernetzung und das Anbinden des anorganischen Materials durchgeführt werden.

Die Erfindung betrifft auch ein wasserabsorbierendes Polysaccharid, welches durch das vorstehend beschriebene Verfahren erhältlich ist.

Das durch das erfindungsgemäße Verfahren erhältliche wasserabsorbierende Polysaccharid zeichnet sich durch ein hervorragendes Absorptions- und Retentionsvermögen für Wasser, wässrige Lösungen und Körperflüssigkeiten aus. Gleichzeitig verfügt es durch die gezielte Vernetzung der Oberfläche über ein deutlich verbessertes Absorptionsvermögen für wässrige Lösungen gegen einen äußeren Druck. Darüber hinaus ist das durch das erfindungsgemäße Verfahren erhältliche wasserabsorbierende Polysaccharid lagerstabil, im wesentlichen frei von bei der Herstellung von Polyacrylaten oftmals anfallenden Restmonomerenanteilen und organischen Lösungsmitteln, nur in geringem Maße in wässrigen Flüssigkeiten löslich und in hohem Maße biologisch abbaubar.

Weiterhin betrifft die vorliegende Erfindung ein durch das erfindungsgemäße Verfahren erhältliches partikuläres, wasserabsorbierendes Polysaccharid, wobei das Polysaccharid mit einem Polyphosphat oder mit Polyphosphorsäure in einer Menge in einem Bereich von 0,001 bis 20 Gew.-%, bevorzugt in einer Menge in einem Bereich von 0,01 bis 10 Gew.-% und besonders bevorzugt in einer Menge in einem Bereich von 0,05 bis 5 Gew.-%, jeweils bezogen auf das Gewicht des Polysaccharids, vernetzt ist.

Zudem kann das wasserabsorbierende Polysaccharid zumindest in dem Oberflächenbereich auch ein Bindemittel aufweisen. Das wasserabsorbierende Polysaccharid weist vorzugsweise anorganische Partikel vorzugsweise in einer Menge im Bereich von 0,001 bis 20 und besonders bevorzugt im Bereich von 0,01 bis 10 Gew.-%, jeweils bezogen auf das wasserabsorbierende Polysaccharid, auf. Unabhängig davon weist das wasserabsorbierende Polysaccharid vorzugsweise Bindemittel vorzugsweise in einer Menge im Bereich von 0,001 bis 20 und besonders bevorzugt im Bereich von 0,01 bis 10 Gew.-%, jeweils bezogen auf das wasserabsorbierende Polysaccharid, auf.

Als Polysaccharide sind dabei diejenigen Polysaccharide bevorzugt, die bereits im Zusammenhang mit dem erfindungsgemäßen Verfahren zur Herstellung eines wasserabsorbierenden Polysaccharids genannt wurden, wobei gleiches auch für anorganische Partikel und für Bindemittel gilt.

In einer bevorzugten Ausführungsform liegt das durch das erfindungsgemäße Verfahren erhältliche, wasserabsorbierende Polysaccharid in einem gemäss ERT 420.1-99 bestimmten mittleren Partikeldurchmesser in einem Bereich von 1 bis 2.000 µm, vorzugsweise in einem Bereich von 100 bis 1.000 µm und besonders bevorzugt in einem Bereich von 150 bis 850 µm vor. Es ist weiterhin bevorzugt, dass mindestens 50 Gew.-%, vorzugsweise mindestens 75 Gew.-% und besonders bevorzugt mindestens 100 Gew.-% des erfindungsgemäßen wasserabsorbierenden Polysaccharids eine durch Siebanalyse bestimmte Teilchengröße im Bereich von 300 bis 600 µm aufweist.

Es ist weiterhin bevorzugt, dass das durch das erfindungsgemäße Verfahren erhältliche, partikuläre, wasserabsorbierende Polysaccharid zumindest eine, vorzugsweise jede, der folgenden Eigenschaften aufweist:
(α1) einen gemäß der hierin beschriebenen Testmethode bestimmten *AUL-Wert* bei einem Druck von 6.21 kPa (0,9 psi) in einem Bereich von 10 bis 22 g/g, besonders bevorzugt in einem Bereich von 12 bis 19 g/g und darüber hinaus bevorzugt in einem Bereich von 14 bis 17 g/g bei einem gemäß der hierin beschriebenen Testmethode bestimmten *CRC-Wert* in einem Bereich von > 15 bis < 20 g/g;
(α2) einen gemäß der hierin beschriebenen Testmethode bestimmten *AUL-Wert* bei einem Druck von 6.21 kPa (0,9 psi) in einem Bereich von 6 bis 20 g/g, besonders bevorzugt in einem Bereich von 8 bis 17 g/g und darüber hinaus bevorzugt in einem Bereich von 10 bis 14 g/g bei einem gemäß der hierin beschriebenen Testmethode bestimmten *CRC-Wert* in einem Bereich von > 20 bis < 25 g/g;
(α3) einen gemäß der hierin beschriebenen Testmethode bestimmten *AUL-Wert* bei einem Druck von 6.21 kPa (0,9 psi) in einem Bereich von 6 bis 15 g/g, besonders bevorzugt in einem Bereich von 7 bis 12 g/g und darüber hinaus bevorzugt in einem Bereich von 8 bis 10 g/g bei einem gemäß der hierin beschriebenen Testmethode bestimmten *CRC-Wert* in einem Bereich von > 25 bis < 30 g/g;
(α4) einen gemäß der hierin beschriebenen Testmethode bestimmten *AUL-Wert* bei einem Druck von 0,9 psi in einem Bereich von 5 bis 12 g/g, besonders bevorzugt in einem Bereich von 6 bis 10 g/g und darüber hinaus bevorzugt in einem Bereich von 7 bis 9 g/g bei einem gemäß der hierin beschriebenen Testmethode bestimmten *CRC-Wert* von > 30 g/g;
Grundsätzlich stellt jede der vorstehenden Ziffern oder eine Kombination daraus eine bevorzugte Ausgestaltung der vorliegenden Erfindung dar. Bevorzugte erfindungsgemäße partikuläre, wasserabsorbierende Polysaccharide sind diejenigen, die durch folgende Eigenschaften oder Eigenschaftskombinationen gekennzeichnet sind: α1, α2, α3, α4, α5, α6, α1α2, α1α3, α1α4, α2α3, α2α4, α3α4, α1α2α3, α1α3α4, α2α3α4, α1α2α3α4.

Es ist weiterhin bevorzugt, dass die durch das erfindungsgemäße Verfahren erhältlichen, partikulären, wasserabsorbierenden Polysaccharide zumindest eine, vorzugsweise jede, der folgenden Eigenschaften aufweisen:
(β1) eine gemäß der hierin beschriebenen Testmethode bestimmte *biologische Abbaubarkeit* von mindestens 40 % in 90 Tagen, vorzugsweise von mindestens 50 % in 90 Tagen und darüber hinaus bevorzugt von mindestens 65 % in 90 Tagen sowie darüber hinaus bevorzugt von mindestens 75 % in 90 Tagen;
(β2) einen gemäß ERT 470.2-99 bestimmten *extrahierbaren Anteil* in einem Bereich von 5 bis 60%, vorzugsweise in einem Bereich von 8 bis 30 % und darüber hinaus bevorzugt in einem Bereich von 10 bis 20 %;
(β3) einen gemäß der hierin beschriebenen Testmethode bestimmten Wert für die *Gel Bed Permeability* in einem Bereich von 1 bis 500, vorzugsweise in einem Bereich von 5 bis 300 und darüber hinaus bevorzugt in einem Bereich von 20 bis 200 × 10-9 cm².

Grundsätzlich stellt jede der vorstehenden Ziffern oder eine Kombination daraus eine bevorzugte Ausgestaltung der vorliegenden Erfindung dar. Bevorzugte erfindungsgemäße partikuläre, wasserabsorbierende Polysaccharide sind diejenigen, die durch folgende Eigenschaften oder Eigenschaftskombinationen gekennzeichnet sind: β1, β2, β3, β1β2, β1β3, β2β3, β1β2β3.

In einer anderen Ausführungsform des durch das erfindungsgemäße Verfahren erhältliche, wasserabsorbierenden Polysaccharids liegt eine gemäß der hierin beschriebenen Testmethode bestimmte *biologische Abbaubarkeit* in einem Bereich von 25 bis 50 % in 45 Tagen und in einem Bereich von mehr als 50 bis 90 % in 90 Tagen, vorzugsweise von mindestens 28 % in 45 Tagen und von mindestens 51 % in 90 Tagen vor.

Es ist weiterhin im Zusammenhang mit den durch das erfindungsgemäße Verfahren erhältlichen, partikulären, wasserabsorbierenden, zumindest teilweise neutralisierten Polysacchariden bevorzugt, dass diese in gequollenem Zustand eine gemäß der hierin beschriebenen Testmethode bestimmte "*Schleimigkeit*" in einem Bereich von 1 bis 3, vorzugsweise in einem Bereich von 1 bis 2 und darüber hinaus bevorzugt von 1 aufweisen.

Es ist weiterhin bevorzugt, dass die durch das erfindungsgemäße Verfahren erhältlichen, partikulären wasserabsorbierenden Polysaccharide einen Innenbereich und einen den Innenbereich umgebenden Aussenbereich aufweisen, wobei der Aussenbereich einen höheren Vernetzungsgrad als der Innenbereich aufweist, so dass sich vorzugsweise eine Kern-Schale-Struktur ausbildet. Die erhöhte Vernetzung im Aussenbereich der vernetzten Polysaccharide wird dabei vorzugsweise durch Nachvernetzung oberflächennaher, reaktiver Gruppen erreicht. Als Nachvernetzer für die Nachvernetzung sind dabei Polyphosphate und Polyphosphorsäure bevorzugt, wobei diejenigen Polyphosphate und Polyphosphorsären besonders bevorzugt sind, die bereits im Zusammenhang mit der ersten Verfahrensstufe des erfindungsgemäßen Verfahrens zur Herstellung wasserabsorbierender Polysaccharide genannte wurden.

Dabei wird als Aussenbereich der Partikel vorzugsweise jedes Volumenelement des Partikels verstanden, dessen Abstand zum Mittelpunkt der Partikel mindestens 75%, vorzugsweise mindestens 85% und besonders bevorzugt mindestens 95% des äußeren Radius der Polymerpartikel beträgt.

Weiterhin betrifft die Erfindung einen Verbund, beinhaltend ein zuvor definiertes wasserabsorbierendes Polysaccharid und ein Substrat. Vorzugsweise sind das erfindungsgemäße wasserabsorbierende Polysaccharid und das Substrat fest miteinander verbunden. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt.

Erfindungsgemäss sind als Verbund Dichtmaterialien, Kabel, absorbierende Cores sowie diese enthaltende Windeln und Hygieneartikel bevorzugt.

Weiterhin betrifft die Erfindung ein Verfahren zur Herstellung eines Verbunds, wobei ein durch das erfindungsgemäße Verfahren erhältliches, wasserabsorbierendes Polysaccharid und ein Substrat und ggf. ein geeignetes Hilfsmittel miteinander in Kontakt gebracht werden. Das in Kontakt bringen erfolgt vorzugsweise durch Wetlaid- und Airlaid-Verfahren, Kompaktieren, Extrudieren und Mischen.

Zudem betrifft die Erfindung einen Verbund, der durch das vorstehende Verfahren erhältlich ist.

Außerdem betrifft die Erfindung die Verwendung des durch das erfindungsgemäße Verfahren erhältlichen, wasserabsorbierenden Polysaccharids oder des zuvor beschriebenen Verbundes in Hygieneprodukten, zur Hochwasserbekämpfung, zur Isolierung gegen Wasser, zur Regulierung des Wasserhaushalts von Böden oder zur Behandlung von Lebensmitteln.

Die Erfindung wird nun anhand von Testmethoden und Beispielen näher erläutert.

### TESTMETHODEN

### BESTIMMUNG DER GEL BED PERMEABILITY (GBP)

Diese Eigenschaft wird nach der in US 6,387,495 B1 offenbarten Testmethode bestimmt.

### BESTIMMUNG DER CENTRIFUGATION RETENTION CAPACITY (CRC)

Diese Eigenschaft wird nach der in EP 0 601 529 B1 offenbarten Testmethode bestimmt.

### BESTIMMUNG DER ABSORPTION UNDER LOAD (AUL)

Diese Eigenschaft wird nach der in EP 0 339 461 B1 offenbarten Testmethode bestimmt, wobei die nachfolgend in den Tabellen genannten Druckbelastungen eingesetzt werden.

### BESTIMMUNG DER SCHLEIMIGKEIT

Hierzu wird das im Rahmen der Bestimmung des CRC erhaltene gequollene Gel bei Tageslicht durch Inaugenscheinnahme bewertet und mit den folgenden Noten nach dem optischen Eindruck belegt. Zur Verdeutlichung wird zudem auf die den einzelnen Noten zugeordneten Bilder verwiesen.

| Note | Optischer Eindruck |
|---|---|
| 1 | einzelne Gelpartikel sind deutlich gegen einander abgegrenzt und haften nicht aneinander. Siehe Bild 1 |
| 2 | einzelne Gelpartikel sind deutlich gegen einander abgegrenzt und haften leicht aneinander. Siehe Bild 2 |
| 3 | Gelschicht mit kaum mehr gegeneinander abgegrenzten Gelpartikeln, die stark aneinander kleben. Siehe Bild 3 |
| 4 | Gelschicht mit nicht mehr erkennbaren Gelpartikeln. Siehe Bild 4 |
| 5 | Kleisterartig fließend. Siehe Bild 5 |

### BESTIMMUNG DER BIOLOGISCHEN ABBAUBARKEIT

Die biologische Abbaubarkeit (Mineralisation) wird durch den Controlled Composting Test (nach ISO 14855, ASTM D5338-92, DIN V54900-2) bestimmt.

### BEISPIELE

### BEISPIEL 1

### 1A) VERFAHRENSSTUFE DES ERFINDUNGSGEMÄßEN VERFAHRENS

Polyphosphorsäure (84 %-ig der Firma Clariant, Deutschland) wird in einer Menge von 0,09 Gew.-%, bezogen auf die Einsatzmenge an Natriumcarboxymethylcellulose, in destilliertem Wasser gelöst und mit Alkalilauge auf einen pH-Wert von 11,5 eingestellt. Die Natriumcarboxymethylcellulose (Cekol^{®} 100.000 der Firma Noviant, Niederlande, mit einem Anteil an wirksamer Substanz von 15 Gew.-%) wird in die Lösung homogen eingeknetet und anschließend gewölft. Das gewölfte Gel wird dann bei Temperaturen von 120°C für 150 Minuten getrocknet und danach auf eine Korngröße in einem Bereich von 850 µm bis 150 µm gemahlen.

Es wird ein Pulver A1 erhalten.

### 1B) VERFAHRENSSTUFE DES ERFINDUNGSGEMÄßEN VERFAHRENS

Eine wässrige Lösung mit einem pH-Wert von 11,0 enthaltend 6 Gew.-%, bezogen auf das Gesamtgewicht der wässrigen Lösung, Polyphosphorsäure als Nachvernetzer, wird mit dem Pulver A1 in einer Menge von 10 Gew.-%, bezogen auf das Gesamtgewicht des Pulvers A1, in Kontakt gebracht. Das beschichtete Vorprodukt wird bei Temperaturen von 130°C für eine Dauer von 50 Minuten erhitzt.

Es wird ein Pulver B1 erhalten.

Die Pulver A1 und B1 waren durch folgende Eigenschaften gekennzeichnet:

**Tabelle 1**

| Pulver | CRC [g/g] | AUL_{2.07 kPa (0,3psi)} [g/g] | AUL_{6.21 kPa (0,9psi)} [g/g] | GBP | Schleimigkeit |
|---|---|---|---|---|---|
| A1 | 30,8 | | | | |
| B1 | 19,2 | 20,3 | 15,6 | 57 | 1 |

### BEISPIEL 2

### 2A) VERFAHRENSSTUFE DES ERFINDUNGSGEMÄßEN VERFAHRENS

Beispiel 1A wurde wiederholt, wobei anstelle von 0,09 Gew.-%, bezogen auf die Einsatzmenge an Natriumcarboxymethylcellulose, 0,1 Gew.-% Polyphosphorsäure (84 %-ig der Firma Clariant, Deutschland) eingesetzt wurde.

Es wird ein Pulver A2 erhalten.

### 2B) VERFAHRENSSTUFE DES ERFINDUNGSGEMÄßEN VERFAHRENS

Beispiel 1B wurde wiederholt, wobei die wässrige Lösung mit einem pH-Wert von 11,0 zusätzlich 0,3 Gew.-%, bezogen auf die Menge an Pulver A2, Aerosil® 200 der Degussa AG, Deutschland enthielt und 5 Gew.-% Polyphosphorsäure, bezogen auf das Gesamtgewicht der wässrigen Lösung, eingesetzt und bei 125°C über 65 Minuten erhitzt wurde.

Es wird ein Pulver B2 erhalten.

Die Pulver A2 und B2 waren durch folgende Eigenschaften gekennzeichnet:

**Tabelle 2**

| Pulver | CRC [g/g] | AUL_{2.07 kPa (0,3psi}) [g/g] | AUL_{6.21 kPa (0,9psi)} [g/g] | GBP | Schleimigkeit |
|---|---|---|---|---|---|
| A2 | 28,5 | | | | |
| B2 | 19,0 | 20,4 | 14,3 | 223 | 1 |

### BEISPIEL 3

### 3A) VERFAHRENSSTUFE DES ERFINDUNGSGEMÄßEN VERFAHRENS

Beispiel 1A wurde wiederholt, wobei anstelle von Cecol^{®} 100.000 nun Cecol^{®} 50.000 eingesetzt wurde.

Es wird ein Pulver A3 erhalten.

### 3B) VERFAHRENSSTUFE DES ERFINDUNGSGEMÄßEN VERFAHRENS

Beispiel 1B wurde wiederholt, wobei die wässrige Lösung mit einem pH-Wert von 11,0 zusätzlich 0,3 Gew.-%, bezogen auf die Menge an Pulver A3, Aerosil® 200 der Degussa AG, Deutschland enthielt. Außerdem wurde bei 130°C über 110 Minuten getrocknet.

Es wird ein Pulver B3 erhalten.

Die Pulver A3 und B3 waren durch folgende Eigenschaften gekennzeichnet:

**Tabelle 3**

| Pulver | CRC [g/g] | AUL_{2.07 kPa (0,3psi)} [g/g] | AUL_{6.21 kPa (0,9psi)} [g/g] | GBP | Schleimigkeit |
|---|---|---|---|---|---|
| A3 | 52,8 | | | | |
| B3 | 20,6 | 20,7 | 14,6 | 142 | 1 |

### BEISPIEL 4

### 4A) VERFAHRENSSTUFE DES ERFINDUNGSGEMÄßEN VERFAHRENS

Beispiel 1A wurde wiederholt, wobei anstelle von 0,09 Gew.-%, bezogen auf die Einsatzmenge an Natriumcarboxymethylcellulose, 0,1 Gew.-% Polyphosphorsäure (84%ig der Firma Clariant, Deutschland) eingesetzt wurde.

Es wird ein Pulver A4 erhalten.

### 4B) VERFAHRENSSTUFE DES ERFINDUNGSGEMÄßEN VERFAHRENS

Beispiel 1B wurde wiederholt, wobei die wässrige Lösung mit einem pH-Wert von 11,0 zusätzlich 0,5 Gew.-%, bezogen auf die Menge an Pulver A4, Sipernat® 22S der Degussa AG, Deutschland enthielt und bei 125°C über 65 Minuten erhitzt wurde.

Es wird ein Pulver B4 erhalten.

Die Pulver A4 und B4 waren durch folgende Eigenschaften gekennzeichnet:

**Tabelle 4**

| Pulver | CRC [g/g] | AUL_{2.07 kPa (0,3psi)} [g/g] | AUL_{6.21 kPa (0,9psi)} [g/g] | GBP | Schleimigkeit |
|---|---|---|---|---|---|
| A4 | 32,9 | | | | |
| B4 | 19,0 | 19,9 | 15,2 | 411 | 1 |

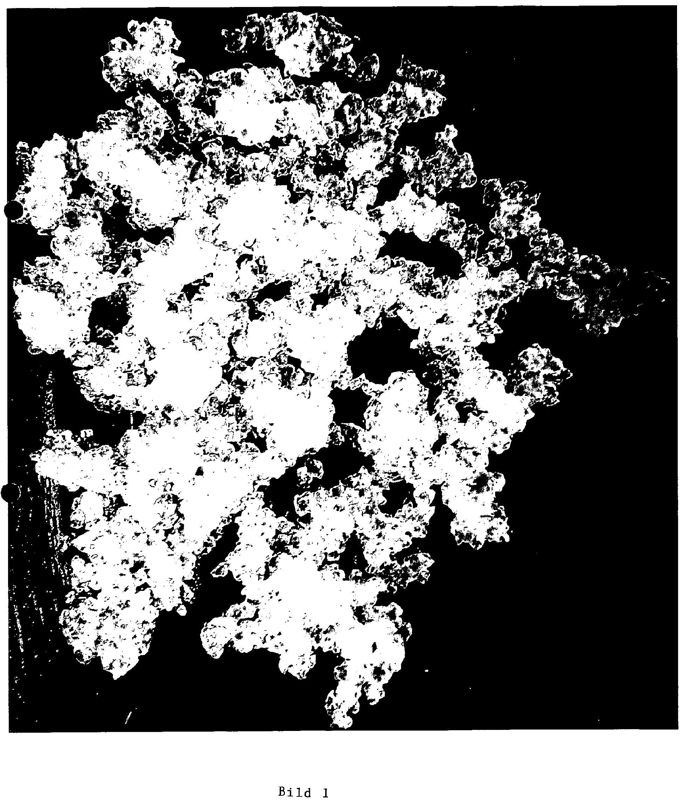

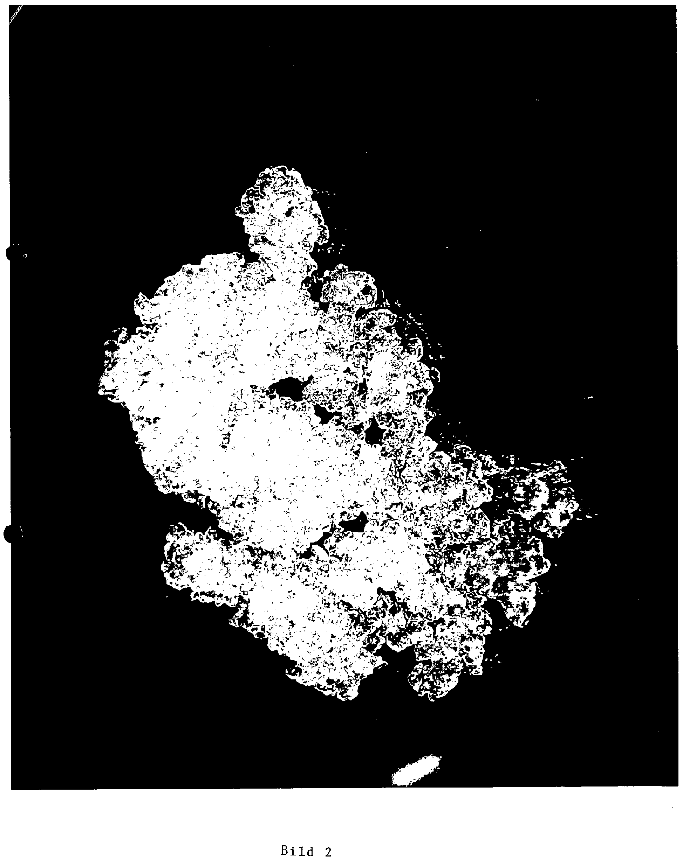

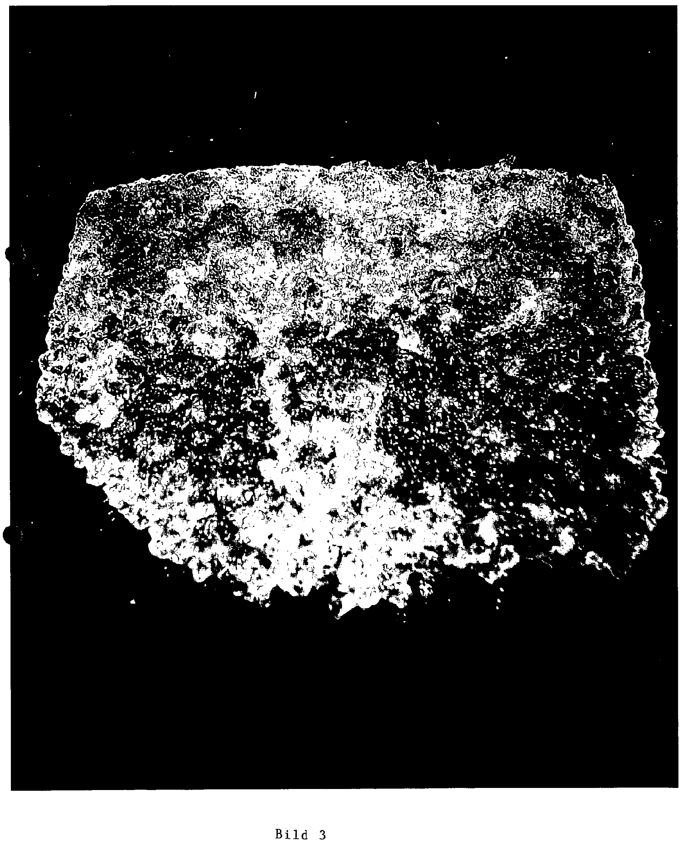

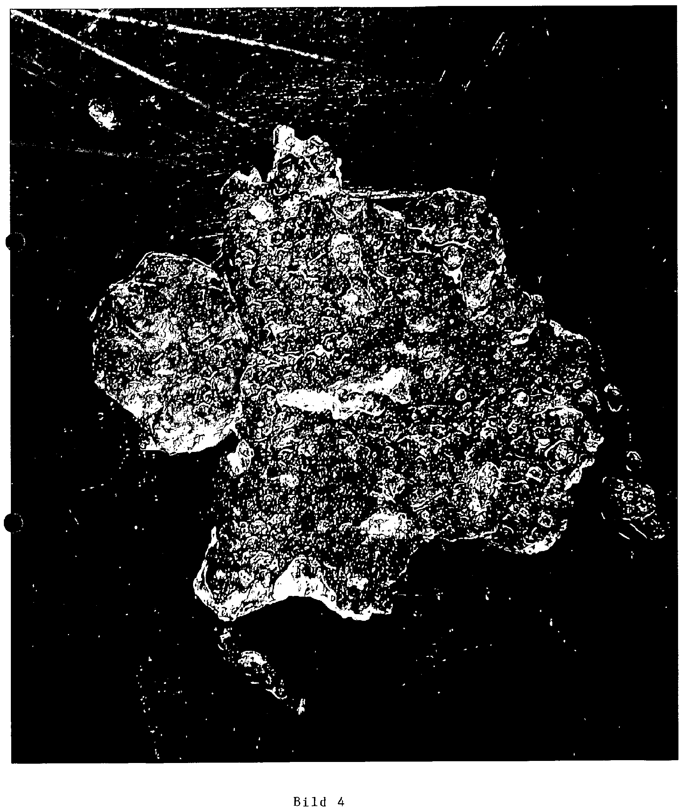

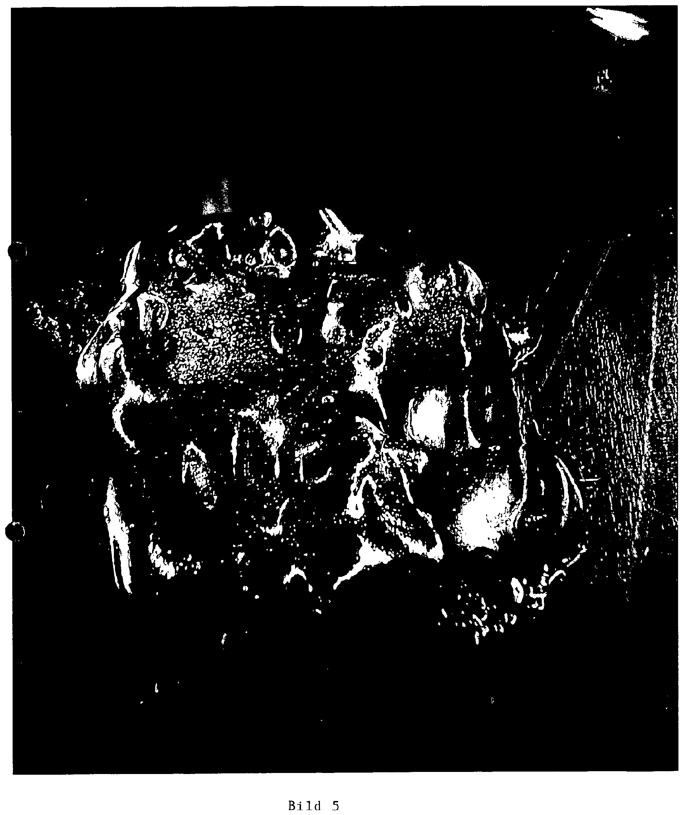

## Patentansprüche

1. Ein Verfahren zur Herstellung eines wasserabsorbierenden Polysaccharids, umfassend die Verfahrensschritte:
- das in Kontakt bringen eines unvernetzten Polysaccharids mit einem Polyphosphat oder mit Polyphosphorsäure als Vernetzungsmittel in Gegenwart von Wasser unter Bildung eines Polysaccharid-Gels;
- Zerkleinern des Polysaccharid-Gels unter Erhalt eines zerkleinerten Polysaccharid-Gels;
- Vernetzen des zerkleinerten Polysaccharid-Gels unter Erhalt eines vernetzten Polysaccharid-Gels, wobei das Polysaccharid-Gel gleichzeitig mit der Vernetzung bis auf einen geringen Restwassergehalt getrocknet wird oder das Polysaccharid-Gel zunächst unter Bedingungen, die nicht zu einem Trocknen des Polysaccharid-gels führen, vernetzt wird und das vernetzte Polysaccharid-Gel anschließend getrocknet wird;
- Mahlen des getrockneten, vernetzten Polysaccharid-Gels unter Erhalt eines partikulären, vernetzten Polysaccharids;
- Nachvernetzen des partikulären, vernetzten Polysaccharids im Außenbereich der Partikel mit einem Nachvernetzungsmittel.

2. Das Verfahren nach Anspruch 1, wobei mindestens das in Kontakt bringen in einem Kneter erfolgt.

3. Verfahren nach Anspruch 2, wobei der Kneter mindestens zwei Knetwellen aufweist.

4. Verfahren nach Anspruch 3, wobei die mindestens zwei Knetwellen eine mindestens teilweise ineinander greifende Kontur besitzen.

5. Verfahren nach Anspruch 3 oder 4, wobei die mindestens zwei Knetwellen einen mindestens zu einer der Knetwellen mindestens teilweise axial verlaufenden Förderkanal ausbilden.

6. Verfahren nach einem der vorhergehenden Ansprüche, wobei das Polysaccharid ein Polycarboxypolysaccharid ist.

7. Verfahren nach Anspruch 6, wobei die Carboxylgruppen des unvernetzten Polycarboxypolysaccharids zu mindestens 50 Mol-% neutralisiert sind.

8. Verfahren nach einem der vorhergehenden Ansprüche, wobei das Vernetzen oder das Trocknen bei einer Temperatur oberhalb von 70 C erfolgt.

9. Verfahren nach einem der vorhergehenden Ansprüche, wobei das in Kontakt bringen des Polysaccharids mit dem Venetzungsmittel in Abwesenheit eines organischen Lösungsmittels erfolgt.

10. Verfahren nach einem der vorhergehenden Ansprüche, wobei das in Kontakt bringen des Vernetzungsmittels bei einem pH-Wert in einem Bereich von 8 bis 12 erfolgt.

11. Verfahren nach einem der vorhergehenden Ansprüche, wobei das in Kontakt bringen des Polysaccharids mit dem Polyphosphat oder mit der Polyphosphorsäure derart erfolgt, dass zunächst das Polyphosphat in Wasser gelöst, in der wässrigen Lösung des Polyphosphats ein pH-Wert in einem Bereich von 8 bis 12 eingestellt und anschliessend die wässrige Lösung des Polyphosphats mit einem unvernetzten Polysaccharid in Kontakt gebracht wird.

12. Verfahren nach einem der vorhergehenden Ansprüche, wobei das Vernetzungsmittel mit dem Polysaccharid in einer Menge in einem Bereich von 0,001 bis 20 Gew. -%, bezogen auf das Gewicht des Polysaccharids, mit dem Polysaccharid in Kontakt gebracht wird.

13. Verfahren nach einem der vorhergehenden Ansprüche, wobei das Polysaccharid einen Salzgehalt von weniger als 20 Gew. -%, bezogen auf das Gesamtgewicht des Polysaccharids, aufweist.

14. Verfahren nach einem der vorhergehenden Ansprüche, wobei das Polyphosphat als Vernetzungsmittel die Zusammensetzung M^{I}ₙ₊₂[PₙO₃ₙ₊₁] M^{I}ₙ [H₂PₙO₃ₙ₊₁] wobei M^{I} ein einwertiges Metall ist und n einen Wert von mindestens 2 aufweist.

15. Verfahren nach einem der vorhergehenden Ansprüche, wobei die Polyphosphorsäure als Vernetzungsmittel die Zusammensetzung Hₙ₊₂PₙO₃ₙ₊₁ oder (HPO3)ₙ aufweist, in der n einen Wert von mindestens 2 besitzt.

16. Verfahren nach einem der vorhergehenden Ansprüche, wobei das Nachvernetzungsmittel in Form einer 0,01 bis 80 Gew.-%igen wässrigen Lösung eingesetzt wird.

17. Verfahren nach Anspruch 16, wobei das Nachvernetzungsmittel ein Polyphosphat oder Polyphosphorsäure ist.

18. Verfahren nach einem der Ansprüche 16 oder 17, wobei das Nachvernetzen der vernetzten Polysaccharide mit dem Nachvernetzungsmittel in Gegenwart anorganischer Partikel erfolgt.

19. Ein wasserabsorbierendes Polysaccharid erhältlich durch ein Verfahren nach einem der vorhergehenden Ansprüche.

20. Ein partikuläres, wasserabsorbierendes Polysaccharid nach Anspruch 19, wobei das Polysaccharid mit einem Polyphosphat oder mit Polyphosphorsäure in einer Menge in einem Bereich von 0,001 bis 25 Gew. -%, bezogen auf das Gewicht des Polysaccharids, vernetzt ist.

21. Partikuläres, wasserabsorbierendes Polysaccharid nach Anspruch 20, wobei das Polysaccharid ein zumindest teilweise neutralisiertes Polycarboxypolysaccharid ist.

22. Partikuläres, wasserabsorbierendes Polysaccharid nach Anspruch 20 oder 21, wobei das Polysaccharid in partikulärer Form mit einem Partikeldurchmesser in einem Bereich von 150 bis 850 µm vorliegt.

23. Partikuläres, wasserabsorbierendes Polysaccharid nach einem der Ansprüche 20 bis 22, wobei das Polysaccharid zumindest eine der folgenden Eigenschaften aufweist: (α1) einen AUL-Wert bei einem Druck von 6.21 kPa (0,9 psi) in einem Bereich von 10 bis 32 g/g bei einem CRC-Wert in einem Bereich von >15 bis < 20 g/g;
(α2) einen AUL-Wert bei einem Druck von 6.21 kPa (0,9 psi) in einem Bereich von 6 bis 20 g/g bei einem CRC-Wert in einem Bereich von > 20 bis < 25 g/g;
(α3) einen AUL-Wert bei einem Druck von 6.21 kPa (0,9 psi) in einem Bereich von 6 bis 15 g/g bei einem CRC-Wert in einem Bereich von > 25 bis < 30 g/g;
(α4) einen AUL-Wert bei einem Druck von 6.21 kPa (0,9 psi) in einem Bereich von 5 bis 12 g/g bei einem CRC-Wert von > 30 g/g.

24. Ein Verbund, beinhaltend ein wasserabsorbierendes, zumindest teilweise neutralisiertes Polysaccharid nach einem der Ansprüche 19 bis 23 und ein Substrat.

25. Ein Verfahren zur Herstellung eines Verbundes, wobei das wasserabsorbierende, zumindest teilweise neutralisierte Polysaccharid nach einem der Ansprüche 19 bis 23 und ein Substrat und gegebenenfalls ein Zusatzstoff miteinander in Kontakt gebracht werden.

26. Ein Verbund erhältlich nach dem Verfahren gemäß Anspruch 25.

27. Verwendung eines wasserabsorbierenden, zumindest teilweise neutralisierten Polysaccharids nach einem der Ansprüche 19 bis 23 oder des Verbundes nach Anspruch 24 oder 26 in Hygieneprodukten, zur Hochwasserbekämpfung, zur Isolierung gegen Wasser, zur Regulierung des Wasserhaushalts von Böden oder zur Behandlung von Lebensmitteln.

## Claims

1. A method for producing a water-absorbing polysaccharide, comprising the method steps of:
- contacting a non-crosslinked polysaccharide with a polyphosphate or with polyphosphoric acid as crosslinking agent in the presence of water, to form a polysaccharide gel;
- comminuting the polysaccharide gel to give a comminuted polysaccharide gel;
- crosslinking the comminuted polysaccharide gel to give a crosslinked polysaccharide gel, the polysaccharide gel simultaneously with the crosslinking being dried to a low residual water content, or the polysaccharide gel first being crosslinked under conditions which do not lead to drying of the polysaccharide gel, and the crosslinked polysaccharide gel being subsequently dried;
- grinding the dried, crosslinked polysaccharide gel to give a particulate, crosslinked polysaccharide;
- post-crosslinking the particulate, crosslinked polysaccharide in the outer region of the particles with a post-crosslinking agent.

2. The method according to Claim 1, wherein at least the contacting takes place in a kneader.

3. Method according to Claim 2, wherein the kneader has at least two kneading shafts.

4. Method according to Claim 3, wherein the at least two kneading shafts possess an at least partially interengaging contour.

5. Method according to Claim 3 or 4, wherein the at least two kneading shafts form a conveying channel which extends at least partially axially at least to one of the kneading shafts.

6. Method according to any of the preceding claims, wherein the polysaccharide is a polycarboxypolysaccharide.

7. Method according to Claim 6, wherein the carboxyl groups of the non-crosslinked polycarboxypolysaccharide are neutralized to an extent of at least 50 mol%.

8. Method according to any of the preceding claims, wherein the crosslinking or the drying takes place at a temperature above 70°C.

9. Method according to any of the preceding claims, wherein the contacting of the polysaccharide with the crosslinking agent takes place in the absence of an organic solvent.

10. Method according to any of the preceding claims, wherein the contacting of the crosslinking agent takes place at a pH in a range from 8 to 12.

11. Method according to any of the preceding claims, wherein the contacting of the polysaccharide with the polyphosphate or with the polyphosphoric acid takes place such that first of all the polyphosphate is dissolved in water, a pH in a range from 8 to 12 is set in the aqueous solution of the polyphosphate, and subsequently the aqueous solution of the polyphosphate is contacted with a non-crosslinked polysaccharide.

12. Method according to any of the preceding claims, wherein the crosslinking agent is contacted with the polysaccharide with the polysaccharide in an amount in a range from 0.001 to 20 wt%, based on the weight of the polysaccharide.

13. Method according to any of the preceding claims, wherein the polysaccharide has a salt content of less than 20 wt%, based on the total weight of the polysaccharide.

14. Method according to any of the preceding claims, wherein the polyphosphate as crosslinking agent has the composition M^{I}ₙ₊₂[PₙO₃ₙ₊₁]M^{I}ₙ[H₂PₙO₃ₙ₊₁], where M^{I} is a monovalent metal and n has a value of at least 2.

15. Method according to any of the preceding claims, wherein the polyphosphoric acid as crosslinking agent has the composition Hₙ₊₂PₙO₃ₙ₊₁ or (HPO3)ₙ, in which n possesses a value of at least 2.

16. Method according to any of the preceding claims, wherein the post-crosslinking agent is used in the form of a 0.01 to 80 wt% strength aqueous solution.

17. Method according to Claim 16, wherein the post-crosslinking agent is a polyphosphate or polyphosphoric acid.

18. Method according to either of Claims 16 and 17, wherein the post-crosslinking of the crosslinked polysaccharides with the post-crosslinking agent takes place in the presence of inorganic particles.

19. A water-absorbing polysaccharide obtainable by a method according to any of the preceding claims.

20. A particulate, water-absorbing polysaccharide according to Claim 19, wherein the polysaccharide is crosslinked with a polyphosphate or with polysphosphoric acid in an amount in a range from 0.001 to 25 wt%, based on the weight of the polysaccharide.

21. Particulate, water-absorbing polysaccharide according to Claim 20, wherein the polysaccharide is an at least partially neutralized polycarboxypolysaccharide.

22. Particulate, water-absorbing polysaccharide according to Claim 20 or 21, wherein the polysaccharide is present in particulate form with a particle diameter in a range from 150 to 850 µm.

23. Particulate, water-absorbing polysaccharide according to any of Claims 20 to 22, wherein the polysaccharide has at least one of the following properties: (α1) an AUL under a pressure of 6.21 kPa (0.9 psi) in a range from 10 to 32 g/g with a CRC in a range from > 15 to < 20 g/g; (α2) an AUL under a pressure of 6.21 kPa (0.9 psi) in a range from 6 to 20 g/g with a CRC in a range from > 20 to < 25 g/g; (α3) an AUL under a pressure of 6.21 kPa (0.9 psi) in a range from 6 to 15 g/g with a CRC in a range from > 25 to < 30 g/g; (α4) an AUL under a pressure of 6.21 kPa (0.9 psi) in a range from 5 to 12 g/g with a CRC of > 30 g/g.

24. An assembly comprising a water-absorbing, at least partially neutralized polysaccharide according to any of Claims 19 to 23 and a substrate.

25. A method for producing an assembly, wherein the water-absorbing, at least partially neutralized polysaccharide according to any of Claim 19 to 23 and a substrate and optionally an additive are contacted with one another.

26. An assembly obtainable by the method according to Claim 25.

27. Use of a water-absorbing, at least partially neutralized polysaccharide according to any of Claims 19 to 23 or of the assembly according to Claim 24 or 26 in hygiene products, for flood control, for insulation against water, for regulating the water content of soils or for treating foodstuffs.

## Revendications

1. Procédé pour la préparation d'un polysaccharide absorbant l'eau, comprenant les étapes de procédé :
- de mise en contact d'un polysaccharide non réticulé avec un polyphosphate ou avec de l'acide polyphosphorique comme agent de réticulation en présence d'eau avec formation d'un gel de polysaccharide ;
- de broyage du gel de polysaccharide avec obtention d'un gel de polysaccharide broyé ;
- de réticulation du gel de polysaccharide broyé avec obtention d'un gel de polysaccharide réticulé, le gel de polysaccharide étant séché en même temps que la réticulation jusqu'à une faible teneur en eau résiduelle ou le gel de polysaccharide étant d'abord réticulé dans des conditions qui ne conduisent pas à un séchage du gel de polysaccharide et le gel de polysaccharide réticulé étant ensuite séché ;
- de broyage du gel de polysaccharide séché, réticulé avec obtention d'un polysaccharide réticulé, particulaire ;
- de postréticulation du polysaccharide réticulé, particulaire dans la zone externe des particules à l'aide d'un agent de postréticulation.

2. Procédé selon la revendication 1, au moins la mise en contact ayant lieu dans un malaxeur.

3. Procédé selon la revendication 2, le malaxeur présentant au moins deux arbres de malaxage.

4. Procédé selon la revendication 3, lesdits au moins deux arbres de malaxage possédant un contour s'engrenant au moins partiellement l'un dans l'autre.

5. Procédé selon la revendication 3 ou 4, lesdits au moins deux arbres de malaxage formant un canal de transport s'étendant de manière au moins partiellement axiale par rapport à au moins un des arbres de malaxage.

6. Procédé selon l'une quelconque des revendications précédentes, le polysaccharide étant un polycarboxypolysaccharide.

7. Procédé selon la revendication 6, les groupes carboxyle du polycarboxypolysaccharide non réticulé étant neutralisés à raison d'au moins 50% en mole.

8. Procédé selon l'une quelconque des revendications précédentes, la réticulation ou le séchage ayant lieu à une température supérieure à 70°C.

9. Procédé selon l'une quelconque des revendications précédentes, la mise en contact du polysaccharide avec l'agent de réticulation ayant lieu en l'absence d'un solvant organique.

10. Procédé selon l'une quelconque des revendications précédentes, la mise en contact de l'agent de réticulation ayant lieu à un pH dans une plage de 8 à 12.

11. Procédé selon l'une quelconque des revendications précédentes, la mise en contact du polysaccharide avec le polyphosphate ou avec l'acide polyphosphorique ayant lieu de manière telle qu'on dissout d'abord le polyphosphate dans l'eau, on règle dans la solution aqueuse du polyphosphate un pH dans une plage de 8 à 12 et on met ensuite en contact la solution aqueuse du polyphosphate avec un polysaccharide non réticulé.

12. Procédé selon l'une quelconque des revendications précédentes, l'agent de réticulation étant mis en contact avec le polysaccharide en une quantité dans une plage de 0,001 à 20% en poids, par rapport au poids du polysaccharide, avec le polysaccharide.

13. Procédé selon l'une quelconque des revendications précédentes, le polysaccharide présentant une teneur en sel inférieure à 20% en poids, par rapport au poids total du polysaccharide.

14. Procédé selon l'une quelconque des revendications précédentes, le polyphosphate présentant, comme agent de réticulation, la composition M^{I}ₙ₊₂[PₙO₃ₙ₊₁]M^{I}ₙ[H₂PₙO₃ₙ₊₁], M^{I} étant un métal divalent et n présentant une valeur d'au moins 2.

15. Procédé selon l'une quelconque des revendications précédentes, l'acide polyphosphorique présentant, comme agent de réticulation, la composition Hₙ₊₂PₙO₃ₙ₊₁ ou (HPO3)ₙ dans lesquelles n présente une valeur d'au moins 2.

16. Procédé selon l'une quelconque des revendications précédentes, l'agent de postréticulation étant utilisé sous forme d'une solution aqueuse à 0,01 jusqu'à 80% en poids.

17. Procédé selon la revendication 16, l'agent de postréticulation étant un polyphosphate ou un acide polyphosphorique.

18. Procédé selon l'une quelconque des revendications 16 ou 17, la postréticulation des polysaccharides réticulés avec l'agent de postréticulation ayant lieu en présence de particules inorganiques.

19. Polysaccharide absorbant l'eau, pouvant être obtenu par un procédé selon l'une quelconque des revendications précédentes.

20. Polysaccharide particulaire, absorbant l'eau, selon la revendication 19, le polysaccharide étant réticulé avec un polyphosphate ou avec de l'acide polyphosphorique en une quantité dans une plage de 0,001 à 25% en poids, par rapport au poids du polysaccharide.

21. Polysaccharide particulaire, absorbant l'eau selon la revendication 20, le polysaccharide étant un polycarboxypolysaccharide au moins partiellement neutralisé.

22. Polysaccharide particulaire, absorbant l'eau, selon la revendication 20 ou 21, le polysaccharide se trouvant sous forme particulaire, présentant un diamètre de particule dans une plage de 150 à 850 µm.

23. Polysaccharide particulaire, absorbant l'eau selon l'une quelconque des revendications 20 à 22, le polysaccharide présentant au moins une des propriétés suivantes :
(α1) une valeur AUL (absorption sous pression) à une pression de 6,21 kPa (0,9 psi) dans une plage de 10 à 32 g/g à une valeur CRC (capacité de rétention à la centrifugeuse) dans une plage de > 15 à < 20 g/g ;
(α2) une valeur AUL à une pression de 6,21 kPa (0,9 psi) dans une plage de 6 à 20 g/g à une valeur CRC dans une plage de > 20 à < 25 g/g ;
(α3) une valeur AUL à une pression de 6,21 kPa (0,9 psi) dans une plage de 6 à 15 g/g à une valeur CRC dans une plage de > 25 à < 30 g/g ;
(α4) une valeur AUL à une pression de 6,21 kPa (0,9 psi) dans une plage de 5 à 12 g/g à une valeur CRC > 30 g/g.

24. Composite, comprenant un polysaccharide absorbant l'eau, au moins partiellement neutralisé, selon l'une quelconque des revendications 19 à 23 et un substrat.

25. Procédé pour la fabrication d'un composite, le polysaccharide absorbant l'eau, au moins partiellement neutralisé selon l'une quelconque des revendications 19 à 23 et un substrat et le cas échéant un additif étant mis en contact les uns avec les autres.

26. Composite pouvant être obtenu selon le procédé selon la revendication 25.

27. Utilisation d'un polysaccharide absorbant l'eau, au moins partiellement neutralisé selon l'une quelconque des revendications 19 à 23 ou du composite selon la revendication 24 ou 26 dans des produits hygiéniques, dans la prévention des inondations, pour l'isolation contre l'eau, pour la régulation du régime des eaux de sols ou pour le traitement d'aliments.
